# EUROPEAN PATENT SPECIFICATION

(11) **EP 2 604 256 B1**
(45) Date of publication and mention of the grant of the patent: **13.04.2016**
(21) Application number: 11193627.4
(22) Date of filing: 14.12.2011
(51) Int. Cl.: A61K 9/50, A61K 31/00, A61K 31/4184, A61K 31/4439, A61K 31/444

(54) **Multi layer coatings**
Mehrschichtige Beschichtungen
Revêtements multicouches

(43) Date of publication of application: 19.06.2013
(73) Proprietor: Hexal AG, 83607 Holzkirchen (DE)
(72) Inventor: Büssing, Christian, 83607 Holzkirchen (DE); Marchesan, Marco, 83607 Holzkirchen (DE); Hummel, Marco, 83607 Holzkirchen (DE)
(74) Representative: Prüfer & Partner mbB Patentanwälte · Rechtsanwälte

(56) References cited:
- WO-A1-2010/041276
- WO-A2-2007/122478
- WO-A2-2008/027993

## Description

### Field of the invention

The present invention belongs to the field of pharmaceutical industry and relates to a process for the preparation of a coated particle. Furthermore, the invention refers to said coated particle itself and a dosage form comprising coated particles, as well as to the use of said dosage form as medicament. Finally, the present invention refers to the use of a combination of a first and a second enteric coating layer for the stabilization of an acid-labile API.

### Description of the background art

Pharmaceutical preparations, compositions or dosage forms, respectively having an enteric coating, are well known in the art. Typical purposes of using enteric coatings include protecting acid sensitive drugs from gastric acid, protecting the gastric mucosa from irritation or damage resulting from drugs that are not well-tolerated when released in the stomach, and modified, controlled or delayed release of a pharmaceutically active ingredient (API).

When designing such enteric coatings, usually the physical and physiological environment in the human digestive tracts should be considered, as well as the passing time in the gastrointestinal tracts. In the gastrointestinal tracts of healthy persons, the physiological pH changes usually from acidic (pH of from about 1.8 to 4.5) to neutral (pH of from about 6.5 to 8.0) from the stomach to the small intestine, and no substantial difference is presumed to exist in the physiological environment between the small intestine and large intestine. The retention time of the preparation in the human stomach is from 0.5 to 10 hours and significantly depends on various factors such as the individual person, a concurrent diet or food intake, and the size of the preparation to be administered. On the other hand, the fluctuations of a passing time through the small intestine are relatively small and the passing time is said to be 3+/-1 hours (h) (Journal of Controlled Release, 2, 27-38 (1985)).

Enteric coatings are known in the art and are typically designed to not/not substantially dissolve in the stomach. An example of commercially available enteric coating polymer is known under the tradename EUDRAGIT^{®} (Evonik, Germany). For instance, EUDRAGIT^{®}S 100 dissolves at a pH value above 7.0 which corresponds to the conditions as present in the colon. This leads to the effect that the enteric coating does not dissolve in the stomach, but in the colon. A further example of an enteric coating polymer is EUDRAGIT^{®} L100-55 or L30D-55, which dissolve at a pH value above 5.5, which means that it dissolves in the duodenum. Further enteric coating examples and further enteric coating polymers exist.

One possible coating technique for applying a coating composition, notably an enteric coating composition, onto a surface of a particle such as e.g. a core, a granule, a dosage formulation or the like is the technique of spray coating. When applying the process of spray coating, the coating composition can e.g. be sprayed onto fluidized particles comprising a pharmaceutically active ingredient (API) of interest.

General principles for applying enteric coatings are for instance described in the Eudragit® Application Guidelines (10th Revised Edition, 06/2008) or elsewhere. According to the Eudragit^{®} Application Guidelines, aqueous systems are more complicated than organic systems because in addition to chemical interactions between anionic and cationic polymers, physical effects may also occur that lead to thickening or coagulation of such mixtures. When providing multi-layer coatings, for example in order to improve the stability of APIs, which are hydrolysed, it is suggested to firstly provide an enteric coating polymer by using an organic solution of enteric polymer and then providing an enteric coating polymer by using an aqueous dispersion of an enteric coating polymer.

It is also known that enteric coating layers comprising enteric polymers with acidic groups may cause degradation of APIs, in particular of acid labile APIs. It is thus common in the art to use intermediate layers, attempting to prevent contact between API and enteric polymer.

In line with this, Shimizu et al. ("Formulation Study for Lansoprazole Fast-disintegrating Tablet. II. Effect of Triethyl Citrate on the Quality of Products", Chem. Pharm. Bull. 51(9) 1029-1035 (2003)) also describes the application of an intermediate layer that is located between a layer comprising the API and an enteric layer comprising enteric polymers with acidic groups. The enteric layer that is located onto the intermediate layer is provided by using an aqueous dispersion.

Despite the above described dosage forms, there is still a need and thus an object for improved coated particles, methods for preparing said coated particles and dosage forms comprising such coated particles.

### Summary of the invention

The present invention provides the following aspects, subject-matters and preferred embodiments, which respectively taken alone or in combination, contribute to solving the object of the present invention:
(1) A process for the preparation of a coated particle comprising the steps of:
   i) providing a core comprising at least one active pharmaceutical ingredient (API),
   ii) providing an intermediate layer onto said core,
   iii) providing a first enteric coating layer onto said intermediate layer by applying an aqueous dispersion of an enteric polymer onto said intermediate layer,
   iv) providing a second enteric coating layer onto said first enteric coating layer by applying an organic solution of an enteric polymer onto said first enteric layer, wherein said organic solution comprises one or more fatty acids and one or more emulsifiers and hydrophilic plasticizer, wherein said hydrophilic plasticizer is propylene glycol, and
   v) obtaining said coated particle,
   wherein the enteric polymer used in step iii) and iv) is an anionic polymer or copolymer.
(2) The process according to item (1), wherein the core comprises at least one acid-labile API.
(3) The process according to item (1) or (2), wherein the type of enteric polymer(s) of the second enteric coating is the same as the type of enteric polymer(s) of the first enteric coating.
(4) The process according to any of the preceding items, wherein the enteric polymer used in step iii) and iv) is an anionic polymer or copolymer with methacrylic acid as functional group.
(5) The process according to any of the preceding items, wherein the enteric polymer used in step iii) and/or iv) is selected from the group consisting of methacrylic acid/ethylacrylate, methacrylic acid/methyl methacrylate, and methacrylic acid/methylacrylate/methyl methacrylate.
(6) The process according to any of the preceding items, wherein at least one API in the core is selected from the group consisting of pravastatin, fluvastatin, atorvastatin, penicillin G, ampicillin, streptomycin, clarithromycin, azithromycin, dideoxyinosine, dideoxyadenosine, dideoxycytosine, digoxin, pancreatin, bupropion, lansoprazole, omeprazole, esomeprazole, pantoprazole, rabeprazole, leminoprazole, hydroxyomeprazole, pariprazole, perprazole, tenatoprazole, diclofenac and pharmaceutically acceptable salts thereof, preferably at least one API is selected from the group consisting of lansoprazole, omeprazole, esomeprazole, pantoprazole, rabeprazole, leminoprazole, hydroxyomeprazole, pariprazole, perprazole and tenatoprazole, or pharmaceutically acceptable salts thereof, more preferably at least one API is selected from the group consisting of lansoprazole, omeprazole, esomeprazole, pantoprazole, rabeprazole, and leminoprazole, or pharmaceutically acceptable salts thereof, even more preferably at least one API is esomeprazole or a pharmaceutically acceptable salt thereof, and most preferably the API is esomeprazole or a pharmaceutically acceptable salt thereof.
(7) The process according to any of items (2) to (6), wherein at least one API in the core is acid-labile and degrades at a pH of 5 or less, preferably the API is selected from the group consisting of pravastatin, fluvastatin, atorvastatin, penicillin G, ampicillin, streptomycin, clarithromycin, azithromycin, dideoxyinosine, dideoxyadenosine, dideoxycytosine, digoxin, pancreatin, bupropion, lansoprazole, omeprazole, esomeprazole, pantoprazole, rabeprazole, leminoprazole, hydroxyomeprazole, pariprazole, perprazole, tenatoprazole, or pharmaceutically acceptable salts thereof, more preferably at least one API is selected from the group consisting of lansoprazole, omeprazole, esomeprazole, pantoprazole, rabeprazole, leminoprazole, hydroxyomeprazole, pariprazole, perprazole and tenatoprazole, or pharmaceutically acceptable salts thereof, even more preferably at least one API is selected from the group consisting of lansoprazole, omeprazole, esomeprazole, pantoprazole, rabeprazole, and leminoprazole, or pharmaceutically acceptable salts thereof, even more preferably at least one API is selected from the group consisting of lansoprazole, omeprazole, esomeprazole, pantoprazole, rabeprazole, and leminoprazole, or pharmaceutically acceptable salts thereof, and most preferably at least one API is esomeprazole or a pharmaceutically acceptable salt thereof.
   In a further preferred embodiment, the API is esomeprazole or a pharmaceutically acceptable salt thereof.
(8) The process according to any of the preceding items, wherein the intermediate layer does not contain an enteric polymer.
(9) The process according to any of the preceding items, wherein the intermediate layer comprises a binding agent, an alkaline stabilizing agent and a lubricant.
(10) The process according to item (9), wherein
   the binding agent is selected from the group consisting of hydroxypropylcellulose, hydroxypropylmethylcellulose, polyvinylpyrrolidone, polyvinylalcohol, polyethyleneglycol, dextrin and pregelatinized starch,
   the alkaline stabilizing agent is selected from the group consisting of magnesium carbonate, magnesium oxide, sodium hydroxide, magnesium hydroxide, magnesium metasilicate aluminate, magnesium silicate aluminate, magnesium silicate, magnesium aluminate, aluminum magnesium hydroxide, calcium carbonate, calcium hydroxide, potassium carbonate, sodium carbonate, sodium hydrogen carbonate and an organic base, and
   the lubricant is selected from the group consisting of magnesium stearate, calcium stearate, talc, light anhydrous silicic acid and hydrated silicon dioxide.
(11) The process according to any of the preceding items, wherein in step iii) the amount of enteric polymer dispersion is in the range of 0.5-20 %, more preferably 5-15 %, based on the weight of the core, and wherein in step iv) the amount of enteric polymer solution for the second enteric coating layer is in the range of 10-100 %, more preferably 20-60 %, based on the weight of the core.
(12) The process according to any of the preceding items, wherein in step iii) the concentration of enteric polymer in the aqueous dispersion is in the range of 10-40 %, preferably 20-30 %, based on the weight of the aqueous polymer dispersion, and wherein in step iv) the concentration of enteric polymer in the organic solution is in the range of 1-20 %, preferably 8-12 %, based on the weight of the organic polymer solution.
(13) The process according to any of the preceding items, wherein in step iii) the aqueous dispersion is applied onto the intermediate layer at a product bed temperature in the range of 25-40°C, preferably 30-35°C, and wherein the organic solution is applied onto the first enteric coating layer at a product bed temperature in the range of 25-40°C, preferably 27-33°C.
(14) The process according to any of the preceding items, wherein in step iii) the atomizing air pressure that is used when applying the aqueous dispersion onto the intermediate layer is in the range of 1.0-6.0 bar, preferably 2.5-3.5 bar, and wherein the atomizing air pressure that is used when applying the organic solution onto the first enteric layer is in the range of 1.0-6.0 bar, preferably 2.5-3.5 bar.
(15) The process according to any of the preceding items, wherein the core is selected from the group consisting of
   i) non-pareilles coated with at least one layer comprising the at least one API; optionally, the non-pareilles is additionally coated with a further layer comprising a further API;
   ii) pellets or granules comprising the at least one API, wherein the pellets or granules are obtained by granulation or extrusion processes; optionally, the pellets or granules additionally comprise a further API; and
   iii) pellets or granules comprising the at least one API, wherein the pellets or granules are obtained by granulation or extrusion processes and additionally comprise one or more coated layers comprising one or more further APIs.
(16) The process according to any of the preceding items, wherein the core has a maximum mean particle size of 1000 µm, preferably the core has a mean particle size in the range of from 400 µm to 500 µm as determined by laser diffraction technology.
(17) The process according to any of the preceding items, wherein the core is a non-pareille, pellet or granule as defined in claim 15, wherein the further API is acid-labile or not acid-labile, preferably the further API is not acid-labile.
(18) The process according to item (17), wherein the further API is selected from the group consisting of salsalate, diflunisal, acetylsalicylic acid, ibuprofen, naproxen, fenoprofen, ketoprofen, flurbiprofen, oxaprozin, loxoprofen, indomethacin, sulindac, etodolac, ketorolac, diclofenac, piroxicam, meloxicam, tenoxicam, droxicam, and lornoxicam, preferably the API is diclofenac.
(19) The process according to any of the preceding items, wherein the aqueous dispersion of an enteric polymer further comprises an emulsifier, preferably an emulsifier selected from the group consisting of polyvinyl pyrrolidone, Na carboxymethylcellulose, polysorbate, more preferably polysorbate 80, and sodium lauryl sulfate.
(20) The process according to any of the preceding items, wherein the aqueous dispersion of an enteric polymer does not contain triethyl citrate.
(21) The process according to any of the preceding items, wherein the organic solution of the enteric polymer further comprises a fatty acid, an emulsifier and optionally a hydrophilic plasticizer;
   wherein the fatty acid preferably contains at least 6 C-atoms, preferably the fatty acid is stearic acid;
   wherein the emulsifier preferably is selected from the group consisting of polyvinyl pyrrolidone, Na carboxymethylcellulose, polysorbate, more preferably polysorbate 80, and sodium lauryl sulfate; and
   the hydrophilic plasticizer is preferably selected from the group consisting of phthalic derivatives, dibutylphthalate, butylphthalylbutylglycolate, propylene glycol, triacetine, silicone oil, diethylphthalate, triethyl citrate, dibutyl sebacate, polyethylene glycol, propylene glycol and combinations thereof, more preferably the hydrophilic plasticizer is propylene glycol.
(22) The process according to any of items (19) to (21), wherein the organic solution of the enteric polymer comprises the hydrophilic plasticizer in an amount of equal to or more than 5 wt.-% and equal to or less than 60 wt.-%, further preferred in an amount of equal to or more than 20 wt.-% and less than or equal to 40 wt.-%, and even further preferred in an amount of equal to or more than 25 wt.-% and equal to or less than 35 wt.-%, based on the amount of the enteric polymer.
(23) The process according to any of items (16) to (19), wherein the organic solution of the enteric polymer contains the fatty acid in an amount of between 5.5 wt.-% and 200 wt.-%, based on the amount of the enteric polymer.
(24) The process according to any of the preceding items, wherein the coated particle comprises only two enteric coating layers in total.
(25) The process according to any of the preceding items, wherein the first and/or second enteric coating is an immediate- or prolonged-release coating.
(26) A process for the preparation of a dosage form, comprising
   a) process for the preparation of a coated particle according to any of items (1) to (25), and
   b) formulating the coated particles of a) into a dosage form.
(27) The process according to item (26), wherein the dosage form the coated particles are formulated into represents a solid oral dosage form, preferably the dosage form is a tablet, preferably a multiple unit tablet, a microtablet or a capsule.
(28) The process according to items (26) or (27), wherein the dosage form the coated particles are formulated into is a multiple unit tablet (MUT).
(29) A coated particle, obtainable and/or obtained according to any one of the processes of items (1)-(25).
(30) A dosage form, obtainable and/or obtained according to any one of the processes of items (26) to (28).
(31) A coated particle comprising, from the inside to the outside:
   i) a core comprising at least one active pharmaceutical ingredient (API),
   ii) an intermediate layer on said core,
   iii) a first enteric coating layer comprising an enteric polymer or combinations of enteric polymers on said intermediate layer,
   iv) a second enteric coating layer comprising an enteric polymer or combinations of enteric polymers on said first enteric coating layer, wherein said second enteric coating layer comprises one or more fatty acids and one or more emulsifiers and hydrophilic plasticizer, wherein said hydrophilic plasticizer is propylene glycol,
      wherein the second enteric coating layer differs from the first enteric coating layer
      (a) in terms of different types of residual solvent that is respectively present in the first and in the second enteric coating layer, the different types of residual solvent being defined by, in comparison with the other type, a relatively higher amount of organic residual solvent in the second enteric coating layer and a relatively higher amount of water residual solvent in the first enteric coating layer, and/or
      (b) in terms of a different compactness of the first and the second enteric coating layer, wherein the first enteric coating layer is less compact than the second enteric coating layer, provided that the enteric polymer/s in the first and in the second enteric coating layer are the same, and/or
      (c) in terms of a different density of the first and the second enteric coating layer, wherein the first enteric coating layer is less dense than the second enteric coating layer, provided that the enteric polymer/s in the first and in the second enteric coating layer are the same,
   wherein the enteric polymer comprised in said first and second enteric coating layer is an anionic polymer or copolymer.
(32) The coated particle according to item (31), characterized by any one or a combination of the following features:
   the core is as defined in any of items (15) to (17);
   the API is as defined in items (6) or (7);
   the intermediate layer is as defined in any of items (8) to (10);
   the enteric polymer in the first and/or second enteric coating layer is an anionic polymer or copolymer, preferably an anionic polymer or copolymer with methacrylic acid as functional group;
   the first enteric coating layer further comprises an emulsifier as defined in item (19); the second enteric coating layer comprises one or more fatty acids and one or more emulsifiersand hydrophilic plasticizer, wherein said hydrophilic plasticizer is propylene glycol.
(33) The coated particle according to item (32), wherein, if the core is as defined in any of items (15) to (17), the further API is as defined in item (17) or (18).
(34) The coated particle according to any of items (31) to (32), wherein the first enteric coating layer does not contain triethyl citrate.
(35) The coated particle according to any of items (31) to (34), wherein the coated particle comprises only two enteric coating layers in total.
(36) The coated particle according to any of items (31) to (35), wherein the first and/or second enteric coating is an immediate- or prolonged-release coating.
(37) A dosage form comprising coated particles according to any of items (31) to (36).
(38) The dosage form according to item (37), wherein the dosage form represents a solid oral dosage form, preferably the dosage form is a tablet, preferably a multiple unit tablet, a microtablet or a capsule.
(39) The dosage form according to items (37) or (38), wherein the dosage form is a multiple unit tablet (MUT).
(40) Use of a dosage form according to item (30), or according to any of items (37) to (39), as a medicament.
(41) Use of a combination of
   a first enteric coating layer formed from an aqueous dispersion comprising an enteric polymer, and
   a second enteric coating layer formed on top of the first enteric coating layer and being formed from an organic solution of an enteric polymer having acidic groups, wherein said organic solution comprises one or more fatty acids and one or more emulsifiers and hydrophilic plasticizer, wherein said hydrophilic plasticizer is propylene glycol
   for the stabilization of an acid-labile API present in a compartment formed underneath the first enteric coating layer, with optional intermediate layer(s) in between said compartment and said first enteric coating layer,
   wherein the enteric polymer is an anionic polymer or copolymer.
(42) Use according to item (43), further for stabilizing a pharmaceutical dosage form containing said acid-labile API against acid use medium and for providing modified release of the acid-labile API.

### Detailed description of the invention

The present invention is now described in more detail by preferred embodiments and examples, which are however presented for illustrative purpose only and shall not be understood as limiting the scope of the present invention in any way.

Within the context of the present invention it has now unexpectedly been found that the combination of a first enteric coating layer, prepared from aqueous dispersion and applied onto an intermediate layer, with a second enteric coating layer, prepared from an organic solution and applied onto the first enteric coating layer, as defined in the claims, provides for an improved stability of an API, in particular an acid-labile API, both against degradation caused by the enteric coating layer as well as against degradation caused by the acidic environment of the stomach. Applying said two enteric coating layers, the stability as well as the level of gastro-resistance of the acid-labile API being present in a particle can be markedly improved compared to the stability and gastro-resistance of an API being present in a particle not comprising the above-specified layers in the right order.
Moreover, by applying the process according to the present invention, in addition to improving the stability and gastro-resistance of the API, in particular of the acid-labile API, it is possible that a modified release of said API can be achieved, if this is desired. With the provision of the above-described combination of enteric coatings, a suitable and highly freely choice of enteric polymers can be made to design a modified, controlled or delayed release of an API of interest, as desired or depending on the case.

Without wishing to be bound by any theory, it is believed that if an enteric coating formed from an organic solution of enteric polymer is used and directly applied on the intermediate coating, some organic solvent diffuses through the intermediate layer, dissolves the API, in particular the acid-labile API, in the core and drags some of the API into the intermediate layer during the drying process. This happens in particular in up-scaling processes, especially in order to increase the spray rate as well as to reduce the process times. The API, in particular the acid-labile API, in the intermediate layer thus comes into contact with the enteric coating layer as well as with the organic solvent, which, in turn, leads to degradation of the API (in particular of the acid-labile API) due to the contact with acidic groups of the enteric polymer, and correspondingly leads to the formation of impurities. When however, firstly an enteric coating layer is applied from an aqueous dispersion, i.e. by limiting or totally omitting organic solvents in the first coating layer, a remarkable further protection of the API (in particular of the acid-labile API) in the core and an enhancement of the protection by the intermediate layer can be achieved.

In combination therewith, subsequently providing, on the first enteric coating layers, a second enteric coating from an organic solution provides for an enhanced stability of the API, in particular of the acid-labile API, against the acidic environment of the stomach.
In the obtained product, the enteric coating layer formed from an organic solution is more compact compared to an enteric coating prepared from aqueous dispersion, and, thus, is different from the enteric coating layer that is formed from an aqueous dispersion.

Thus, it has been found that a previously unmet combination of effects can be achieved by the present invention, including avoiding degradation of an (acid-labile) API, additionally providing, by applying the process according to the present invention, a modified release of the (acid-labile) API. A particular advantage is provided by the use of the same type of enteric polymer, preferably the enteric polymers are the same, in the first and second enteric layer. By using the same type of enteric polymer, in particular by using the same enteric polymer, in the first and in the second enteric layer, possible incompatibilities that may arise between different types of enteric polymers, or different enteric polymers, can be avoided, thereby further contributing to an increased stability of the (acid-labile) API. Further, by using the same type of enteric polymer, in particular by using the same enteric polymer, in the first and in the second enteric layer, the stability of the (acid-labile) API as well as the stability of the dosage form, e.g. regarding the dissolution behaviour, can be improved.

The present invention thus relates to a process for the preparation of a coated particle comprising the steps of:
i) providing a core comprising at least one active pharmaceutical ingredient (API),
ii) providing an intermediate layer onto said core,
iii) providing a first enteric coating layer onto said intermediate layer by applying an aqueous dispersion of an enteric polymer onto said intermediate layer,
iv) providing a second enteric coating layer onto said first enteric coating layer by applying an organic solution of an enteric polymer onto said first enteric layer, wherein said organic solution comprises one or more fatty acids and one or more emulsifiers and hydrophilic plasticizer, wherein said hydrophilic plasticizer is propylene glycol, and
v) obtaining said coated particle,
wherein the enteric polymer used in step iii) and iv) is an anionic polymer or copolymer.

Herein, the term "coated particle" refers to a particle comprising an API-containing core and coated layers including for example an intermediate layer and enteric coating layers on said core. The core can for example be an API-containing homogenous pellet or can be a non-pareille (starter) which is itself coated with one or more API-containing layer(s).

The coated particle described herein comprises a core comprising at least one API. Additionally preferred, the core comprises at least one acid-labile API. Within the meaning of this invention, the term "acid-labile API" refers to APIs that comprise functional groups that are susceptible to the presence of protons. Due to the presence of these acid-labile functional groups, the APIs degrade at a pH of 5 or less to a substantial degree.

Preferably the acid-labile API is selected from the group consisting of pravastatin, fluvastatin, atorvastatin, penicillin G, ampicillin, streptomycin, clarithromycin, azithromycin, dideoxyinosine, dideoxyadenosine, dideoxycytosine, digoxin, pancreatin, bupropion, lansoprazole, omeprazole, esomeprazole, pantoprazole, rabeprazole, and leminoprazole, hydroxyomeprazole, pariprazole, perprazole, tenatoprazole, and pharmaceutically acceptable salts thereof. Further preferred, the acid-labile API is a prazole, preferably a prazole selected from the group consisting of lansoprazole, omeprazole, esomeprazole, pantoprazole, rabeprazole, and leminoprazole, hydroxyomeprazole, pariprazole, perprazole, tenatoprazole, and pharmaceutically acceptable salts thereof. As mentioned above, acid-labile APIs can also degrade upon contact with acid functional groups of an enteric polymer.

In general, the core can contain one or more APIs that is/are not acid-labile or that is/are acid-labile. In one aspect, the core comprises at least one acid-labile and at least one non acid-labile API. Examples of various medicinal substances (APIs) include, without being limited to, antifungal agents like e.g. fluconazol; non steroidal anti-inflammatory drugs, such as antipyretic analgesic anti-inflammatory agents (for example, indomethacin, aspirin, diclofenac sodium, ketoprofen, ibuprofen, mefenamic acid, azulene, phenacetin, isopropylantipyrine, acetaminophen, bendzac, phenylbutazone, flufenamic acid, sodium salicylate, salicylamide, sasapyrine, etodolac and the like); steroidal anti-inflammatory agents (for example, dexamethasone, hydrocortisone, prednisolone, triamcinolone and the like); antiulcer agents (for example, 5-amino salicylic acid, ecabet sodium, enprostil, sulpiride, cetraxate hydrochloride, gefarnate, irsogladine maleate, cimetidine, ranitidine hydrochloride, famotidine, nizatidine, roxatidine acetate hydrochloride and the like); coronary vasodilators (for example, nifedipine, isosorbide dinitrate, diltiazem hydrochloride, trapidil, dipyridamole, dilazep dihydrochloride, verapamil, nicardipine, nicardipine hydrochloride, verapamil hydrochloride and the like); peripheral vasodilators (for example, ifenprodil tartrate, cinepazet maleate, cyclandelate, cinnarizine, pentoxifylline and the like); antibiotics (for example, ampicillin, amoxicillin, cephalexin, erythromycin ethylsuccinate, bacampicillin hydrochloride, minocycline hydrochloride, chloramphenicol, tetracycline, erythromycin, ceftazidime, cefuroxime sodium, aspoxicillin, ritipenem acoxil hydrate and the like); synthetic antimicrobials (for example, nalidixic acid, piromidic acid, pipemidic acid trihydrate, enoxacin, cinoxacin, ofloxacin, norfloxacin, ciprofloxacin hydrocloride, sulfamethoxazole trimethoprim and the like); antiviral agents (for example, aciclovir, ganciclovir and the like); antispasmodics (for example, propantheline bromide, atropine sulfate, oxapium bromide, timepidium bromide, butylscopolamine bromide, trospium chloride, butropium bromide, N-methyl scopolamine methyl sulfate, methyloctatropine bromide and the like); antitussives (for example, tipepidine hibenzoate, methylephedrine hydrochloride, codeine phosphate, tranilast, dextromethorphan hydrobromide, dimemorfan phosphate, clobutinol hydrochloride, fominoben hydrochloride, benproperine phosphate, eprazinone hydrochloride, chlophedianol hydrochloride, ephedrine hydrochloride, noscapine, pentoxyverine citrate, oxeladine citrate, isoaminil citrate and the like); expectorants (for example, bromhexine hydrochloride, carbocysteine, ethylcysteine hydrochloride, methylcysteine hydrochloride and the like); bronchodilators (for example, theopylline, aminophylline, disodium cromoglycate, procaterol hydrochloride, trimetoquinol hydrochloride, diprophylline, salbutamol sulfate, clorprenaline hydrochloride, formoterol fumarate, orciprenalin sulfate, pirbuterol hydrochloride, hexoprenaline sulfate, bitolterol mesilate, clenbuterol hydrochloride, terbutaline sulfate, mabuterol hydrochloride, fenoterol hydrobromide, methoxy phenamine hydrochloride and the like); cardiacs (for example, dopamine hydrochloride, dobutamine hydrochloride, docarpamine, denopamine, caffeine, digoxin, digitoxin, ubidecarenon and the like); diuretics (for example, furusemide, acetazolamide, trichlormethiazide, methyclothiazide, hydrochlorothiazide, hydroflumethiazide, ethiazide, cyclopenthiazide, spironolactone, trimterene, fluorothiazide, piretanide, mefruside, ethacrynic acid, azosemido, clofenamide and the like); muscle relaxants (for example, chlorphenesin carbamate, tolperisone hydrochloride, eperisone hydrochloride, tizanidine hydrochloride, mephenesin, chlorzoxazone, phenprobamate, methocarbamol, chlormezanone, pridinol mesylate, afloqualone, baclofen, dantrolene sodium and the like); brain metabolism improvers (for example, nicergoline, meclofenoxate hydrochloride, taltirelin and the like); minor tranquilizers (for example, oxazolam, diazepam, clotiazepam, medazepam, temazepam, fludiazepam, meprobamate, nitrazepam, chlordiazepoxide and the like); major tranquilizers (for example, sulpiride, clocapramin hydrochloride, zotepine, chlorpromazine, haloperidol and the like); beta-blockers (for example, bisoprolol fumarate, pindolol, propranolol hydrochloride, carteolol hydrochloride, metoprolol tartrate, labetalol hydrochloride, acebutolol hydrochloride, bufetolol hydrochloride, alprenolol hydrochloride, arotinolol hydrochloride, oxprenolol hydrochloride, nadolol, bucumolol hydrochloride, indenolol hydrochloride, timolol maleate, befunolol hydrochloride, bupranolol hydrochloride and the like); antiarrhythmic agents (for example, procainamide hydrochloride, disopyramide, ajmaline, quinidine sulfate, aprindine hydrochloride, propaphenone hydrochloride, mexiletine hydrochloride, azimilide hydrochloride and the like); antipodagrics (for example, allopurinol, probenecid, colchicine, sulfinpyrazone, benzbromarone, bucolome and the like); anticoagulants (for example, ticlopidine hydrochloride, dicumarol, warfarin potassium, (2R,3R)-3-acetoxy-5-[2-(dimethylamino)ethyl]-2,3-dihydro-8-methyl-2-(4-methylphenyl)-1,5-benzothiazepine-4(5H)-on maleate and the like); thrombolytic agents (for example, methyl(2E,3Z)-3-benzyliden-4-(3,5-dimethoxy- alpha -methylbenzyliden)-N-(4-methylpiperazine-1-yl)succinamate hydrochloride and the like); agents for liver disease (for example, (+/-)r-5-hydroxymethyl-t-7-(3,4-dimethoxyphenyl)-4-oxo-4,5,6,7-tetrahydrobenzo[b]furan-c-6-carbonate lactone and the like); antiepileptics (for example, phenytoin, sodium valproate, metharbital, carbamazepine and the like); antihistamines (for example, chlorpheniramine maleate, clemastine fumarate, mequitazine, alimemazine tartrate, cycloheptazine hydrochloride, bepotastine besilate and the like); antiemetics (for example, difenidol hydrochloride, metoclopramide, donperidone, betahistine mesylate, trimebutine maleate and the like); hypotensive agents (for example, dimethylaminoethylreserpinate dihydrochloride, recinnamine, methyldopa, prazosin hydrochloride, bunazosin hydrochloride, clonidine hydrochloride, budralazine, urapidil, N-[6-[2-[(5-bromo-2-pyrimidinyl)oxy]ethoxy]-5-(4-methylphenyl)-4-pyrimidinyl]-4-(2-hydroxy-1,1-dimethylethyl)benzenesulfonamide sodiumsalt and the like); agents for hyperlipemia (for example, pravastatin sodium, fluvastatin sodium and the like); sympathomimetic agents (for example, dihydroergotamine mesylate, isoproterenol hydrochloride, etilefrine hydrochloride and the like); oral antidiabetics (for example, glibenclamide, tolbutamide, glymidine sodium and the like); oral carcinostatic agents (for example, marimastat and the like); alkaloid narcotic (for example, morphine, codeine, cocaine and the like); vitamins (for example, vitamin B1, vitamin B2, vitamin B6, vitamin B12, vitamin C, folic acid and the like); agents for treatment of pollakisuria (for example, flavoxate hydrochloride, oxybutynin hydrochloride, terodiline hydrochloride and the like); angiotensin converting enzyme inhibitors (for example, imidapril hydrochloride, enalapril maleate, alacepril, delapril hydrochloride and the like); and the like.

In a preferred embodiment, the at least one API is acid-labile, wherein the core preferably additionally contains (a) further API(s) that is/are not acid-labile. A preferred non acid-labile API is diclofenac.

In one further aspect, the core comprises two or at least two APIs that are acid-labile. In another aspect, the core comprises at least one acid-labile API in combination with at least one non acid-labile API. In another aspect, the core comprises one acid-labile and one non acid-labile API. The acid-labile and non acid-labile API(s) can e.g. be selected from the APIs indicated above.

The core comprises a suitable amount of the API(s), for example at least 5 wt.-% or at least 10 wt.-%, preferably an amount in a range of 20-80 wt.-%, also preferred an amount in a range of 20-60 wt.-%, also preferred 20-40 wt.-%, based on the coated particle. The core is described in more details elsewhere herein.

Onto said core, an intermediate layer is provided. The intermediate layer separates the API-containing core from the first enteric coating layer. The intermediate layer will be described in more details elsewhere herein.

The core with the intermediate layer thereon is provided with at least two enteric coating layers, a first enteric coating layer and a second enteric coating layer. Although further enteric coatings can be applied, it has been found within the context of the present invention that the first and second enteric coating described herein provide for improved stability during storage as well as during passage of the coated particle through the stomach compared to particles not comprising the inventive enteric coating layers. By keeping the number of coating layers low, the process for preparing said coated particle is most economic. Thus, providing two enteric coating layers is of economic advantage. Consequently, the use of only two enteric coating layers is preferred.

As used in the context of the present invention as defined in the claims, the term enteric polymer refers to anionic polymer or copolymer.

The first and second enteric coating layers can comprise the same type of enteric polymer (i.e. the enteric polymers have the same type of monomers but not necessarily the same molecular weight), or the same enteric polymer (or polymers, if more than one enteric polymer is used in one layer), or different types of enteric polymers. It is therefore possible that the first enteric coating layer comprises the same type of, or the same, enteric polymer (or mixture of enteric polymers) as the second enteric coating layer. Also, the first enteric coating layer can comprise an enteric polymer (or mixture of enteric polymers) other than that of the second enteric coating layer. The use of the same enteric polymer/s or type of enteric polymer/s, or mixtures thereof, can additionally contribute to an enhanced stability of the (acid-labile) API that is present in the core.

In a preferred embodiment, the enteric polymer of the second enteric coating is of the same type, preferably is the same enteric polymer, as the enteric polymer of the first enteric coating. The enteric polymers being of the same type, or even being the same, may reduce the risk of incompatibilities between the components of the first and the second enteric coating layer, which, in turn, may even further contribute to an enhanced stability of the API being present in the core.

Suitable enteric polymers are listed elsewhere herein. If more than one enteric polymer is used, the enteric polymers can be understood as a mixture of enteric polymers. If further enteric coating layers (i.e. in addition to the first and the second enteric coating layer) are applied, those may also contain said enteric polymer(s). By definition, an enteric polymer or mixture of enteric polymers does not dissolve at a pH below 5.5. The enteric polymer to be used according to the present invention can be one polymer or a mixture of polymers. In the following, the polymer or mixture of polymers, respectively, will be referred to as the "enteric polymer". The term "polymer" as used herein refers to homopolymers as well as to copolymers. The enteric polymer or enteric coating, respectively, does not dissolve under conditions as present in the stomach of a human or animal body. The dissolution properties of an enteric polymer or enteric coating can be determined by using e.g. in-vitro tests such as dissolution and disintegration tests. Such tests are for instance described in the European Pharmacopoeia (chapter 2.9.3., 7th Edition 2011 (7.2)) e.g. for prolonged-release dosage forms and delayed-release dosage forms. For example with regard to delayed-type dosage forms, these in-vitro tests are usually carried out in two stages. In the first stage, the dosage form is tested in an acid phase (HCl buffer), followed by a second stage in a suitable buffer (e.g. pH 6.8, phosphate buffer). Moreover, the dissolution profile of enteric coatings can also be determined by a method as described below (e.g. as described in Example 5). Preferably the enteric polymer/enteric coating fulfils the requirements for enteric coatings as defined in the European Pharmacopoeia, chapter 2.9.3. (7th Edition 2011 (7.2)), in particular as defined for delayed-type dosage forms. Within the meaning of the present invention, the term "modified release" denotes that the escape of the drug (API) from the dosage form, e.g. the tablet, has been modified in some way.

According to the present invention, the enteric polymer is an anionic polymer or copolymer, preferably at least one selected from the group consisting of enteric cellulose derivatives, enteric acrylic copolymers, enteric maleic copolymers, enteric polyvinyl derivatives and shellac. Even further preferred, the enteric polymer is an anionic polymer with methacrylic acid as a functional group. Further preferred the enteric polymer is an anionic copolymer selected from the group consisting of methacrylic acid/ethylacrylate, methacrylic acid/methyl methacrylate, and methacrylic acid/methylacrylate/methyl methacrylate.

In one embodiment, the anionic polymer or mixture of anionic polymers is used in combination with an methacrylate copolymer having neutral ester groups (e.g. R=COOCH₃ or R=COOC₄H₉) or trimethylammonioethyl groups (R=COOCH₂CH₂N⁺(CH₃)₃Cl). Methacrylate copolymers having a neutral ester are insoluble and pH-independent and can be used to provide a time controlled release dosage form, wherein e.g. after dissolution of the anionic polymer a matrix of the insoluble polymer remains and the medicinal substance can be washed out of this matrix.

In a preferred embodiment, the enteric polymer/enteric coating layers dissolve(s) above a pH of 5.5. A dosage form comprising such enteric coating layers will therefore release the API directly after leaving the stomach, e.g. in the duodenum.
In another embodiment of the invention, the enteric polymer/enteric coating layers dissolve(s) at a pH of between 5.5 and 7.0.
In another embodiment, the enteric polymer/coating layers dissolve(s) at a pH above 7.0. In another embodiment, the enteric polymer/enteric coating layers dissolve(s) at a pH of between 5.5 and 6.5, most preferred at a pH of between 5.5 and 6.0.

Preferred enteric cellulose derivatives according to the present invention are hydroxypropylmethylcellulose acetate succinate, hydroxypropylmethylcellulose phthalate, hydroxymethylethylcellulose phthalate, cellulose acetate phthalate, cellulose acetate succinate, cellulose acetate maleate, cellulose benzoate phthalate, cellulose propionate phthalate, methylcellulose phthalate, carboxymethylethylcellulose or ethylhydroxyethylcellulose phthalate.

Suitable hydroxymethylethylcellulose phthalates are also sold unter the tradename HP50^{®} and HP55^{®} (Shin-Etsu, Japan).

Preferred enteric acrylic copolymers are styrene/acrylic acid copolymer, methyl acrylate/ acrylic acid copolymer, methyl acrylate/ methacrylic acid copolymer, butyl acrylate/ styrene / acrylic acid copolymer, methacrylic acid/ methyl methacrylate copolymer, methacrylic acid/ ethylacrylate copolymer or methyl acrylate/ methacrylic acid/ octyl acrylate copolymer, or methacrylic acid/methylacrylate/methyl methacrylate.

Suitable anionic polymers or copolymers with methacrylic acid as a functional group are sold unter the tradename EUDRAGIT^{®} (Evonik, Germany): EUDRAGIT^{®} L100-55 (sold in powder form; dissolution above pH 5.5), EUDRAGIT^{®} L30 D-55 (sold as aqueous dispersion, 30%; dissolution above pH 5.5), EUDRAGIT^{®} L100 (sold in powder form; dissolution above pH 6.0) or EUDRAGIT^{®} L12,5 (sold as organic solution, 12.5%; dissolution above pH 6.0), EUDRAGIT^{®} S100 (sold in powder form; dissolution above pH 7.0), EUDRAGIT^{®} S12,5 (sold as organic solution, 12.5%; dissolution above pH 7.0) or EUDRAGIT^{®} FS 30D (sold as aqueous dispersion, 30%; dissolution above pH 7.0).

Suitable methacrylate copolymers having neutral ester groups are also sold under the tradename EUDRAGIT^{®} (Evonik, Germany): EUDRAGIT^{®} NE 300/400 /NM 30D and methacrylate copolymers having trimethylammonioethyl groups are sold under the tradename EUDRAGIT^{®} (Evonik, Germany): EUDRAGIT^{®} RL100, EUDRAGIT^{®} RL PO/ RS PO, EUDRAGIT^{®} RL 30D/RS 30D, and EUDRAGIT^{®} RL12.5/ RS12,5.

Preferred enteric maleic copolymers are vinylacetate/ maleic acid anhydride copolymer, styrene/ maleic acid anhydride copolymer, styrene/ maleic acid monoester copolymer, vinylmethylether/ maleic acid anhydride copolymer, ethylene/ maleic acid anhydride copolymer, vinylbutylether/ maleic acid anhydride copolymer, acrylonitrile/ methyl acrylate/ maleic acid anhydride copolymer or butyl acrylate/ styrene/ maleic acid anhydride copolymer.

Preferred enteric polyvinyl derivatives are polyvinyl alcohol phthalate, polyvinylacetal phthalate, polyvinyl butylate phthalate or polyvinylacetoacetal phthalate.

If enteric polymers in the first and second enteric coating layer are of the same type, both layers contain a polymer having the same monomers but not necessarily the same molecular weight. If enteric polymers are the same, the polymer is identically used for both layers.

In a further embodiment of the process described herein, in step iii) the amount of enteric polymer dispersion for the first enteric coating layer can preferably be in the range of 0.5-20 %, more preferably 5-15 %, based on the weight of the core, and wherein in step iv) the amount of enteric polymer solution for the second enteric coating layer can preferably be in the range of 10-100 %, more preferably 20-60 %, based on the weight of the core.

If the core comprises a non-pareille (starter), the amount of enteric polymer dispersion for the first enteric coating layer can preferably be in the range of 1-20 %, more preferably 10-15 %, based on the weight of the non-pareille, and the amount of enteric polymer in the second enteric coating layer can preferably be in the range of 20-100 %, more preferably 40-60 %, based on the non-pareille.

In a further embodiment of the process described herein, one or more fatty acids and one or more emulsifiers are present in the organic solution which is used for providing the second enteric coating layer. In addition, the organic solution contains a hydrophilic plasticizer, wherein said hydrophilic plasticizer is propylene glycol. Additionally preferred, the aqueous dispersion which is used for providing the first enteric coating layer comprises an emulsifier. Further preferred, the emulsifier is present in both the organic solution and aqueous dispersion.

The use of fatty acids and hydrophilic plasticizers in enteric coating compositions effectively avoids the formation of agglomerates in the coating process and further enhances the elasticity and flexibility of the resulting enteric coating. Unlike conventional glidants (sometimes also referred to as separating agents or as lubricants) like talc or glycerol monostearate, the use of fatty acids as separating agents (sometimes also referred to as release agents) beneficially does not decrease the elasticity of the coating and does not lead to fissuring, thereby avoiding the release of API into the stomach which could lead to irritation of the mucosa of the stomach.

As a constituent of the enteric coatings according to the present invention, any fatty acid can be used. Preferably such a fatty acid has at least 6 carbon atoms, further preferred between 6 and 22 carbon atoms. Particularly preferred, such a fatty acid comprises 8 to 20 carbon atoms, still particularly preferred 10 to 18 carbons and especially preferred 12 to 18 carbon atoms. The fatty acid can be saturated or unsaturated, preferably the fatty acid is a saturated acid. Preferably the saturated fatty acid is selected from the group consisting of stearic acid, lauric acid, myristic acid, palmitic acid or behenic acid, particular preferred, stearic acid is used. According to the present invention, the above-mentioned fatty acids may be employed not only alone but also in a mixture of two or more thereof.

Preferably the amount of fatty acid to be used according to the invention is in an amount of equal to or more than 5.5 wt.-% and equal to or less than 200 wt.-%, preferably in an amount of equal to or more than 10 wt.-% and equal to or less than 150 wt.-%, further preferred in an amount of equal to or more than 10 wt.-% and equal to or less than 100 wt.-%, still further preferred in an amount of equal to or more than 10 wt.-% and equal to or less than 50 wt.-%, particularly in an amount of equal to or more than 20 wt.-% and equal to or less than 50 wt.-%, more particularly in an amount of equal to or more than 20 wt.-% and equal to or less than 40 wt.-%, further preferred in an amount of equal to or less than 30 wt.-% and equal to or less than 40 wt.-%, and most preferred in an amount of equal to or less than 35 wt.-% and equal to or less than 40 wt.-%, based on the amount of the enteric polymer.

The plasticizer which can be used according to the present invention can be a pharmaceutically acceptable plasticizer which can be used in combination with an enteric polymer to provide an enteric coating. In general, plasticizers are low-molecular substances that should exhibit a dissolution profile similar to that of the enteric polymer. Furthermore, they should be homogenously distributable in the enteric polymer. The specific interaction between the plasticizer and the enteric polymer, the plasticizers among each other and the enteric polymers among each other leads to an increased flexibility of the enteric polymer, which is also reflected in a change of physical properties of the enteric polymer. For instance, the glass transition temperature and brittleness decrease with increasing flexibility.

Plasticizers are known in the art and may, in principle, be hydrophilic or hydrophobic molecules. Within the meaning of the present invention, the plasticizer is the hydrophilic plasticizer propylene glycol. In a preferred embodiment, the hydrophilic plasticizer according to the present invention interacts with a fatty acid also being present in the enteric coating composition or in the enteric coating, respectively. The fatty acid, which can also be regarded as a hydrophobic plasticizer, interacts with the hydrophilic plasticizer, thereby e.g. increasing the flexibility and/or elasticity of the enteric coating and diminishing the brittleness of the enteric coating. Furthermore, by adding the hydrophilic plasticizer according to the present invention to an enteric coating composition, a relatively fast, yet controllable release of the API being present in the dosage form can be achieved. It is assumed that this is reached by synergistic interaction of the hydrophilic plasticizer with the enteric polymer.

In the present invention, the hydrophilic plasticizer is propylene glycol.
In a further embodiment, no triethyl citrate is present in the enteric coating layers.

According to the present invention, the plasticizer is used in an amount of equal to or more than 5 wt.-% and equal to or less than 60 wt.-%, further preferred in an amount of equal to or more than 20 wt.-% and less than or equal to 40 wt.-%, and even further preferred in an amount of equal to or more than 25 wt.-% and equal to or less than 35 wt.-%, based on the amount of the enteric polymer. In a preferred embodiment, the plasticizer is used in an amount of about 30 wt.-% based on the amount of enteric polymer.

In addition, the enteric coating layers may contain an emulsifier. Suitable emulsifiers are e.g. non ionic surfactants like e.g. polysorbates, e.g. polysorbate 80 or 20, polyvinyl pyrrolidone, Na carboxymethylcellulose, or sodium lauryl sulfate. Preferably, the emulsifier is a polysorbate.

In a preferred embodiment, the first enteric coating applied by using an aqueous dispersion of an enteric polymer comprises an emulsifier as described herein, preferably the emulsifier is polysorbate 80 or sodium lauryl sulfate, more preferably, the emulsifier is polysorbate 80, and the second enteric coating applied by using an organic solution comprises an emulsifier as described herein, preferably the emulsifier is polysorbate 80 or sodium lauryl sulfate, more preferably, the emulsifier is polysorbate 80. In addition, the second enteric coating comprises a fatty acid as described herein, preferably stearic acid. The second enteric coating additionally comprises the hydrophilic plasticizer propylene glycol.

The combination of the described amount of plasticizer with the fatty acid leads to an enhanced elasticity and/or flexibility of the enteric coating. This is in particular advantageous in case enteric coated particles are formulated into dosage forms by using a compression process, where high pressures are applied onto the coated particles. This is for instance the case if the enteric coated particles are compressed into tablets, in particular in multiple unit tablets. In case the flexibility of the coating is too low, fissuring can occur. The damaged film is not able to protect the API during the passage through the stomach and/or to protect the stomach from the potential irritating effects of the API.

The coated particle comprises one or more intermediate layer(s) on the core, i.e. the intermediate layer is present between the core containing an (acid-labile) API and the first enteric coating layer. The intermediate layer does not contain acid functional groups. Preferably, the intermediate layer does not comprise an enteric polymer. The intermediate layer can comprise a binding agent, an alkaline stabilizing agent and/or a lubricant. In order to enhance stability of an API, preferably of an acid-labile API, in a preferred embodiment, an alkaline stabilizing agent is used. The amount of the intermediate layer in dry state being present on the coated particle suitably is 5 - 45 wt.-%, preferably 20 - 40 wt.-%, or even preferred 30 - 35 wt.-%, based on the weight of the starter particle, i.e. the particle that is subjected to coating with the intermediate layer.

Within the meaning of the present invention, the term "coated particle" denotes the core that is provided with an intermediate layer and enteric coatings comprising the first and the second enteric coating as described elsewhere herein. In one embodiment, said coated particle comprises only two enteric coating layers in total, said first and second enteric coating. The coated particle can be formulated into a dosage form.

The binding agent can e.g. be selected from the group consisting of hydroxypropylcellulose, hydroxypropylmethylcellulose, polyvinylpyrrolidone, polyvinylalcohol, polyethyleneglycol, dextrin and pregelatinized starch, the alkaline stabilizing agent can e.g. be selected from the group consisting of magnesium carbonate, magnesium oxide, sodium hydroxide, magnesium hydroxide, magnesium metasilicate aluminate, magnesium silicate aluminate, magnesium silicate, magnesium aluminate, aluminum magnesium hydroxide, calcium carbonate, calcium hydroxide, potassium carbonate, sodium carbonate, sodium hydrogen carbonate and an organic base, and the lubricant can e.g. be selected from the group consisting of magnesium stearate, calcium stearate, talc, light anhydrous silicic acid and hydrated silicon dioxide.

The organic base can e.g. be tris(hydroxymethyl)aminomethane or 1-deoxy-1-(methylamino)-D-glucitol.

Within the invention, the coating layers can be provided according to well known methods. When preparing enteric coating layers, for instance the methods set forth in the Eudragit® Application Guidelines (10th Revised Edition, 06/2008) can be considered.

The solvent can be used for preparing organic coating solutions. In one embodiment, the organic solvent that is present in the organic coating solution of the enteric polymer can e.g. be ethanol, acetone or isopropanol, or mixtures thereof. The organic solvent can be in admixture with water, wherein the maximum amount of water in the organic solution is such that still an enteric polymer solution can be provided. In one embodiment, the organic solution can comprise up to 50 wt.-%, preferably less than 25 wt.-%, further preferred less than 10 wt.-%, even further preferred less than 5 wt.-%, of water, based on the total amount of solvents. Preferably, the organic coating solutions do not contain any water.

The aqueous dispersion can, besides water, contain some amounts of organic solvents, for instance the organic solvents listed above, wherein the amount of organic solvents in the aqueous dispersion is such that in combination with the enteric polymer still an aqueous dispersion is provided. In a further embodiment, the aqueous dispersion contains less than 25%, preferably less than 10%, further preferred less than 5%, by weight of organic solvents, based on the total amount of solvents.

Preferably, in step iii) the concentration of enteric polymer in the aqueous dispersion is in the range of 10-40 %, preferably 20-30 %, based on the weight of aqueous dispersion, and in step iv) the concentration of enteric polymer in the organic solution is in the range of 1-20 %, preferably 8-12 %, based on the weight of organic solution.

In the process according to the present invention, the enteric coating layers are applied onto the preceding layers, e.g. the intermediate layer and the first enteric coating layer, by any suitable method that is known to a person skilled in the art, e.g. by using spray coating, in particular by a fluid bed coating process. Suitable conditions that may be used for spray coating, such as the product bed temperature or the atomizing air pressure, are known to a person skilled in the art. In a preferred embodiment, the enteric coating layers are applied by using spray coating.

In a preferred embodiment, in step iii), the aqueous dispersion is applied onto the intermediate layer at a product bed temperature in the range of 25-40°C, more preferably 30-35°C, and the organic solution is applied onto the first enteric coating layer at a product bed temperature in the range of 25-40°C, more preferably 27-33°C.

Preferably, the atomizing air pressure that is used when applying the aqueous dispersion onto said intermediate layer is in the range of 1.0-6.0 bar, more preferably 2.5-3.5 bar, and the atomizing air pressure that is used when applying the organic solution onto said first enteric layer is in the range of 1.0-6.0 bar, more preferably 2.5-3.5 bar.

Within the present invention, the intermediate layer and enteric coating layers can be applied onto any suitable core comprising at least one API, preferably an acid-labile API, that is known to a person skilled in the art.

In particular, the core can e.g. comprise a non-pareille, tablet, granule, grain or pellet. Furthermore, the core can consist of a coated non-pareille, a coated granule, coated grains or a coated pellet. The core can have any shape, for example it has a spherical shape. The non-pareille can e.g. be a sugar sphere having a mean diameter of about 250 to about 1000 microns (µm).

Preferably, the core is selected from the group consisting of
i) non-pareilles coated with at least one layer comprising the at least one API that is preferably acid-labile; optionally, the non-pareilles is additionally coated with a further layer comprising one or more further API;
ii) pellets or granules comprising the at least one API that is preferably acid-labile, wherein the pellets or granules are obtained by granulation or extrusion processes; optionally, the pellets or granules additionally comprise one or more further API; and
iii) pellets or granules comprising the at least one API that is preferably acid-labile, wherein the pellets or granules are obtained by granulation or extrusion processes and additionally comprise one or more coated layers comprising one or more further APIs.

The core can be prepared by suitable known methods. For instance, the non-pareilles, pellets or granules can be coated by suitable known coating methods such as coating, spray coating, extrusion procedures and granulation.
The pellets or granules can also be prepared by any suitable method that is known to a person skilled in the art, such as granulation (such as wet granulation, dry granulation, layering granulation and impregnate granulation) or extrusion.

In wet granulation, according to a conventional method, granulation and/or grading may be carried out by granulating with an agitating granulator, a high speed mixer granulator and the like after a binder solution has been added to a mixture in which an API is mixed with various additives for the preparation, or by employing an oscillating granulating machine after kneading and adding a binding solution to a mixture of an API and various additives for the preparation. Further, granulation can be carried out by employing a fluidized bed granulator, agitation fluidized bed granulator and the like with spraying a binder solution to a fluidizing mixture of an API and various additives for preparations.

In dry granulation, a mixture of an API and various additives may be granulated by employing a roller compactor, a roll granulator and the like.

In case a layering granulation is carried out, an API (if necessary, together with other pharmaceutical additives) is added to a rolled inert carrier, while a binder solution which has to be attached onto the carrier is sprayed using a centrifugal fluidized bed coater and the like.

Examples of the inert carrier include, for instance, crystallines of sugars or inorganic salts such as crystalline of lactose, microcrystalline cellulose, crystalline of sodium chloride, a spherical granulated material such as the spherical granulated material of crystalline cellulose (available from Asahi Chemical Industry Co., Ltd.; Trade-name: Avicel SP), the spherical granulated material of crystalline cellulose and lactose (available from Freund Industrial Co., Ltd.; Trade-name: Nonpareil NP-5, and Nonpareil NP-7), the spherical granulated material of refined sugar (available from Freund Industrial Co., Ltd.; Trade-name: Nonpareil-103), and the spherical granulated material of lactose and alpha -starch.

In impregnating granulation, usually suitable concentrations of API solution are mixed with a porous carrier in order to keep the API solution into the pore portion of the carrier, and then the mixture may be dried in order to remove the solvent.

Examples of a porous carrier include, for instance, aluminum magnesium metasilicate (available from Fuji Chemical Industry Co., Ltd.; Trade-name: NEUSILIN), calcium silicate (available from Eisai Co., Ltd.; Trade-name: FLORITE) and the like.

In an embodiment according to the present invention, the core can have any suitable size; preferably, the core has a mean particle size from about 250 µm to about 750 µm, preferably from about 400 to about 500 µm, as determined by laser diffraction technology, e.g. with Malvern 2000 Master sizer.

In another embodiment according to the present invention, the non-pareille can have any suitable size; preferably, the non-pareille has a mean particle size from about 250 µm to about 750 µm, preferably from about 400 to about 500 µm, as determined by laser diffraction technology, e.g. with Malvern 2000 Master sizer.

As already described elsewhere herein, the core can be a non-pareille, pellet or granule, comprising at least one further API (i.e. an API besides and in addition to the (acid-labile) API). This further API can be an acid-labile or non acid-labile API, preferably the further API is non acid-labile.

In a further embodiment, the at least one further API is not contained in the same coated layer as the (acid-labile) API or the at least one further API is not contained in the pellet or granule containing the at least one (acid-labile) API.

The further API is selected from the group consisting of non-steroidal anti-inflammatory drugs (NSAID). Non-steroidal anti-inflammatory drugs as well as processes for their preparation are well known in the art, for example from E. Friderichs et al. "Analgesics and Antipyretics", Ullmann's Encyclopedia of Industrial Chemistry, Sixth Edition, Wiley-VCH Verlag GmbH, Germany 2000, pages 1-22 and H. Buschmann, T. Christoph, E. Friderichs, C. Maul, B. Sundermann, "Analgesics - From Chemistry and Pharmacology to Clinical Application", 2002, Part II, Wiley-VCH Verlag, Germany. In one embodiment, the NSAID is selected from the group consisting of acemetacin, acetaminophen, acetylsalicylic acid, azulene, bendzac, bufexamac, diclofenac, diclofenac-sodium, diflunisal, dipyrone (metamizol), metamizol-sodium, ethenzamide, etodolac, etofenamate, flufenamic acid, flurbiprofen, ibuprofen, indomethacin, isopropylantipyrine, isoxicam, kebuzone, ketoprofen, ketorolac, lonazolac, lornoxicam, meclofenamic acid, mefenamic acid, mofebutazone, nabumetone, naproxen, (+)-ibuprofen, (-)- ibuprofen, (+)-naproxen, niflumic acid, oxaprozin, oxyphenbutazone, phenacetin, phenylbutazone, piroxicam, propyphenazone, salicylamide, sasapyrine, sulindac, tenoxicam, tiaprofenic acid, SC560, sulphasalazine, tolmetin, and the like. In a preferred embodiment, the NSAID is selected from the group consisting of acetylsalicylic acid, ibuprofen, ketoprofen, and indomethacin.

In a further embodiment, the further API is selected from the group consisting of salsalate, diflunisal, acetylsalicylic acid, ibuprofen, naproxen, fenoprofen, ketoprofen, flurbiprofen, oxaprozin, loxoprofen, indomethacin, sulindac, etodolac, ketorolac, diclofenca, piroxicam, meloxicam, tenoxicam, droxicam, lornoxicam, preferably the further API is diclofenac.

If the core comprises an (acid-labile) API as further API, the core can contain an alkalizing agent (also referred to herein as alkaline stabilizing agent) in order to further enhance stability of the API. The alkaline stabilizing agent can be selected from the group as described elsewhere herein.

In a further embodiment according to the present invention, the first and/or second enteric coating is a controlled- or prolonged-release coating. Within the meaning of the present invention, the term "immediate-release" denotes that the total API being contained in a dosage form comprising the coated particles (and being present in the core of said coated particles) is released "immediately" after the passaging through the stomach, e.g. within a period of 0.5 h after passaging through the stomach. The term "prolonged-release" denotes that the total API being contained in a dosage form comprising the coated particles (and being present in the core of said coated particles) is released after passage through the stomach, but over a prolonged period of e.g. more than 0.5 h, e.g. over more than 1 or 2 hours. Immediate-release and prolonged-release can be achieved by the addition of certain enteric polymers or excipients that are known in the art.

The release profile of dosage forms can be determined by using the protocol of the European Pharmacopeia, 7th Edition 2011 (7.2), Monograph Tablets, Chapter 2.9.3, Dissolution Test for Solid Dosage Forms using an Agilent 1260 Infinity, diode array detector.

In a further embodiment, it is also possible to include a coloring agent in the enteric coating and enteric coating composition, respectively. Examples of the coloring agent include for instance food dyes, lake dyes, caramel, carotenes, annatto, cochineal, iron dioxides and the like, and an opaque coloring agent, OPALUX, mostly made of a lake dye and syrup. Examples of food dyes include food aluminum lakes such as food red No.2, No.3, yellow No.4, No. 5, green No. 3, blue No. 1, No. 2 and purple No.1; annatto (natural color derived from Bixa orellana); carmine (aluminum carminate); pearl essence which mainly consists of guanine, and the like.

It is additionally possible, and in certain cases preferred, to include a masking agent in the enteric coating and enteric coating composition, respectively. Examples of a masking agent include titanium dioxide, precipitated calcium carbonate, dibasic calcium phosphate, calcium sulfate, and the like.

It is additionally preferred to include a lubricant in the enteric coating and enteric coating composition, respectively. Examples of the lubricant encompass magnesium stearate, talc, synthetic magnesium silicate, fine grained silicon oxide, and sodium stearyl fumarate.

In addition, other excipients can be included in the enteric coating according to the present invention.

The present invention also refers to a process for the preparation of a dosage form, comprising
a) process for the preparation of a coated particle as described elsewhere herein, and
b) formulating the coated particles of a) into a dosage form.
The dosage form the coated particles are formulated into represents a solid oral dosage form, preferably the dosage form is a tablet, preferably a multiple unit tablet, a microtablet or a capsule. In a preferred embodiment, the dosage form the coated particles are formulated into is a multiple unit tablet (MUT). The formulation of the dosage forms can be carried out by any suitable method that is known to a person skilled in the art.

The present invention further refers to a coated particle, obtainable and/or obtained according to the process as described herein, as well as to a dosage form obtainable and/or obtained according to the process as described herein.

The invention also refers to a coated particle comprising, from the inside to the outside:
i) a core comprising at least one active pharmaceutical ingredient (API) that is preferably acid-labile,
ii) an intermediate layer on said core,
iii) a first enteric coating layer comprising an enteric polymer or combinations of enteric polymers on said intermediate layer,
iv) a second enteric coating layer comprising an enteric polymer or combinations of enteric polymers on said first enteric coating layer, wherein said second enteric coating layer comprises one or more fatty acids and one or more emulsifiers and hydrophilic plasticizer, wherein said hydrophilic plasticizer is propylene glycol,
wherein the second enteric coating layer differs from the first enteric coating layer
(a) in terms of different types of residual solvent that is respectively present in the first and in the second enteric coating layer, the different types of residual solvent being defined by, in comparison with the other type, a relatively higher amount of organic residual solvent in the second enteric coating layer and a relatively higher amount of water residual solvent in the first enteric coating layer, and/or
(b) in terms of a different compactness of the first and the second enteric coating layer, wherein the first enteric coating layer is less compact than the second enteric coating layer, provided that the enteric polymer/s in the first and in the second enteric coating layer are the same, and/or
(c) in terms of a different density of the first and the second enteric coating layer, wherein the first enteric coating layer is less dense than the second enteric coating layer, provided that the enteric polymer/s in the first and in the second enteric coating layer are the same, wherein the enteric polymer comprised in said first and second enteric coating layer is an anionic polymer or copolymer.

With regard to the different types of residual solvent that is present in the first and in the second enteric coating layer under item (a) above, in a preferred embodiment the first coating layer only contains insignificant amounts of organic solvent, more preferably the first coating layer is free or entirely free from organic solvent. Conversely, alternatively or in addition thereto, in a preferred embodiment the second enteric coating layer only contains insignificant amounts of water, more preferably the second coating layer is free or practically entirely free from water. The residual solvent that is present in the respective enteric coating layer can be determined by any suitable method that is known to a person skilled in the art. Such methods can for instance be based on spectroscopic methods, such as Raman spectroscopic methods.

With regard to the different compactness of the first and the second enteric coating layer under item (b) above, in a preferred embodiment the components of the second enteric coating layer, prepared by organic solution, are arranged in a more compact structure, as e.g. determined by suitable compactness-related methods. Such methods are known to a person skilled in the art, e.g. visual observation under appropriate magnification.
As another suitable indication, the different compactness may be illustrated by a phenomenon that the components of the second enteric coating layer are arranged within a relative smaller space compared to the arrangement of the components of the first enteric coating layer which is prepared by aqueous dispersion. In order to exclude inter-variation by other factors, it is further preferred that the specified different compactness is determined only by way of the influence of using either aqueous medium or using organic solvent during preparation of the first and second enteric coating layers, respectively. It is thus particularly preferred that the enteric polymer/s in the first and in the second enteric coating layer are the same. In a further embodiment, solid components that are relevant for the determination of compactness (e.g. plasticizer, fatty acid, surfactant) in the first and in the second enteric coating layer are the same or are respectively absent.

With regard to the different density of the first and the second enteric coating layer under item (c) above, in a preferred embodiment the second enteric coating layer comprises more mass per volume. In other words, in the second enteric coating layer, a higher quantity of components is present per unit volume, compared to the first enteric coating layer. The mass per volume, as well as the quantity of components, can for instance be determined by suitable density-related methods. Such methods are known to a person skilled in the art, e.g. by isotope-based methods. In order to exclude inter-variation by other factors, it is further preferred that the specified different density is determined only by way of the influence of using either aqueous medium or using organic solvent during preparation of the first and second enteric coating layers, respectively. It is thus particularly preferred that the enteric polymer/s in the first and in the second enteric coating layer are the same. In a further embodiment, all solid components that are relevant for the determination of density (e.g. plasticizer, fatty acid, surfactant) in the first and in the second enteric coating layer are the same.

The core, API, intermediate layer, first and second enteric coating layer have the characteristics and can be prepared as described in detail above.

The present invention further refers to a dosage form comprising coated particles as described herein. Preferably, the dosage form represents a solid oral dosage form, preferably the dosage form is a tablet, preferably a multiple unit tablet, a microtablet or a capsule. Further preferred, the dosage form is a multiple unit tablet (MUT).

The invention also refers to the use of a dosage form according to the invention as a medicament. For example, dosage forms comprising prazoles, in particular in combination with other APIs like diclofenac, can e.g. be in the treatment of dyspepsia, peptic ulcer disease (PUD), gastroesophageal reflux disease (GORD/GERD), laryngopharyngeal reflux (LPR) and Zollinger-Ellison syndrome.

The present invention further refers to the use of a combination of a first enteric coating layer formed from an aqueous dispersion comprising an enteric polymer, and a second enteric coating layer formed on top of the first enteric coating layer and being formed from an organic solution of an enteric polymer having acidic groups, wherein said organic solution comprises one or more fatty acids and one or more emulsifiers and hydrophilic plasticizer, wherein said hydrophilic plasticizer is propylene glycol, for the stabilization of an acid-labile API present in a compartment formed underneath the first enteric coating layer, with optional intermediate layer(s) in between said compartment and said first enteric coating layer, wherein the enteric polymer is an anionic polymer or copolymer.

In a further embodiment, the present invention refers to the use of a combination of a first enteric coating layer formed from an aqueous dispersion comprising an enteric polymer, and a second enteric coating layer formed on top of the first enteric coating layer and being formed from an organic solution of an enteric polymer having acidic groups, wherein said organic solution comprises one or more fatty acids and one or more emulsifiers and hydrophilic plasticizer, wherein said hydrophilic plasticizer is propylene glycol, for the stabilization of an acid-labile API present in a compartment formed underneath the first enteric coating layer, with optional intermediate layer(s) in between said compartment and said first enteric coating layer, and further for stabilizing a pharmaceutical dosage form containing said acid-labile API against acid use medium and for providing modified release of the acid-labile API, wherein the enteric polymer is an anionic polymer or copolymer.

### Methods

### Method for determining particle size:

The following method can be used for determining the mean particle size, e.g. the mean particle size of the core (or non-pareille) used in the present invention: European Pharmaocopeia, 7th Edition 2011 (7.2), Chapter 2.9.12: "Sieve analysis", or European Pharmaocopeia, 7th Edition 2011 (7.2), Chapter 2.9.31: Particle size analysis by laser light diffraction using Malvern Mastersizer 2000 and a dry dispersion system.

### Solubility screening of API in different solvents

Solubility screening can be carried out according to European Pharmaocopeia, 7th Edition 2011 (7.2), Chapter 1.4: "Monographs-Solubility" by using a UV-photometer ProStar 350 Photo Diode Array Detector.

### Method for determining the gastro-resistance of the API containing dosage form/API containing composition

In order to determine the gastro-resistance of an API containing dosage form/API containing composition over time, a little modified method, based on European Pharmaocopeia, 7th Edition 2011 (7.2), Chapter 2.9.3 for delayed release dosage forms, was carried out. The main parameters are the paddle speed (at 100 rpm), duration (4 h) and acid stage (0.05M Na-Acetate buffer pH 5.0) of pretreatment and buffer stage at pH 6.8.

### Method for determining the stability of the API

Stability of an API can be derived from the Drug Master Files (DMF)-information of the API-manufacturer or can be determined according to ICH Q1AR2 (stability testing as a function of packaging material and storage conditions) e.g. at accelerated conditions: 40°C ± 2°C/75% RH ± 5% RH.

### Description of the figures

**Fig**. **1****:** This figure shows Formulation A, comprising a starter, a drug layer comprising acid-labile API, an intermediate layer (protection layer) and a gastro-resistant (enteric coating) layer (coated via aqueous dispersion).

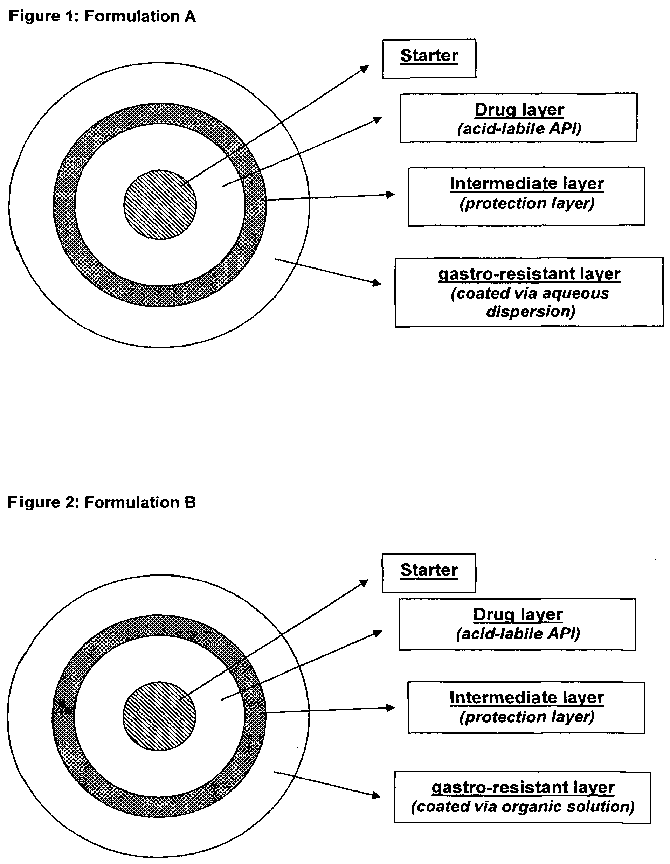

**Fig. 2****:** This figure shows Formulation B, comprising a starter, a drug layer comprising acid-labile API, an intermediate layer, and a gastro-resistant (enteric coating) layer (coated via organic solution).

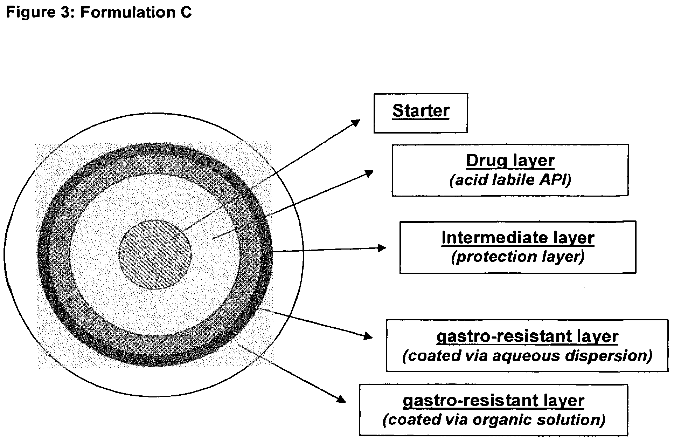

**Fig. 3****:** This figure shows Formulation C, comprising a starter, a drug layer comprising acid-labile API, an intermediate layer, a gastro-resistant (enteric coating) layer (coated via aqueous dispersion; first enteric coating layer), and a gastro-resistant (enteric coating) layer (coated via organic solution; second enteric coating layer).

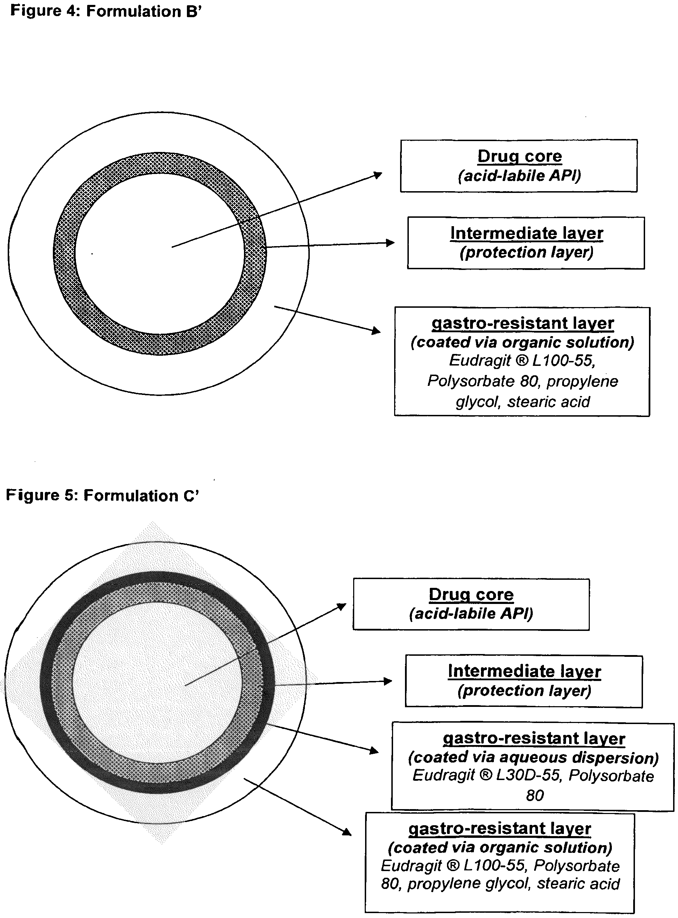

**Fig. 4**: This figure shows Formulation B', comprising a drug core comprising acid-labile API, an intermediate layer, and a gastro-resistant (enteric coating) layer (coated via organic solution), comprising Eudragit® L100-55, Polysorbate 80, propylene glycol, and stearic acid.
**Fig. 5**: This figure shows Formulation C', comprising a drug core comprising acid-labile API, an intermediate layer, a first gastro-resistant (enteric coating) layer (coated via aqueous dispersion, comprising Eudragit ® L30D-55, and Polysorbate 80) and a second gastro-resitant layer (coated via organic solution, comprising Eudragit ® L100-55, Polysorbate 80, propylene glycol, and stearic acid).

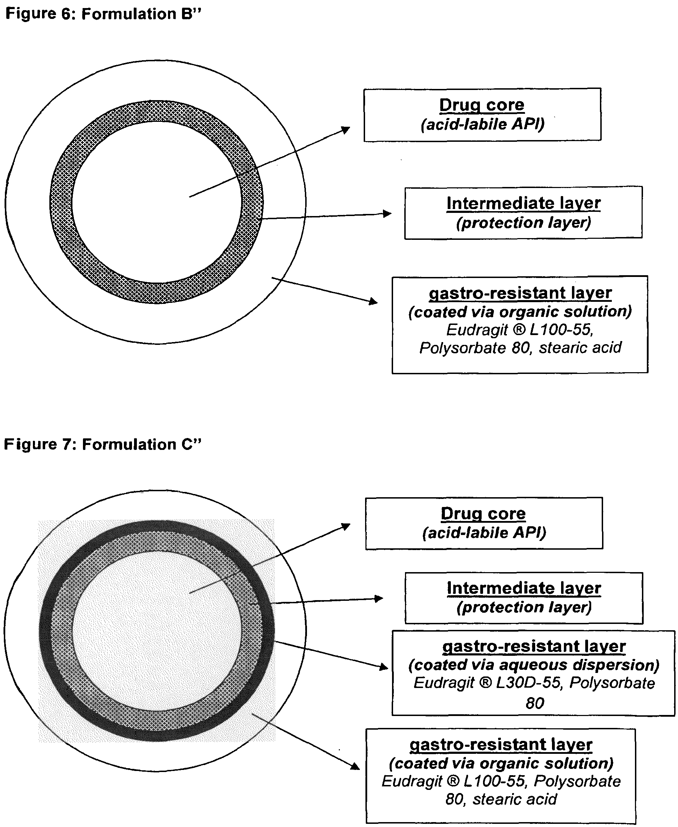

**Fig. 6**: This figure shows Formulation B", comprising a drug core comprising acid-labile API, an intermediate layer, and a gastro-resistant (enteric coating) layer (coated via organic solution, comprising Eudragit ® L100-55, Polysorbate 80, and stearic acid).
**Fig. 7**: This figure shows Formulation C", comprising a drug core comprising acid-labile API, an intermediate layer, a first gastro-resistant (enteric coating) layer (coated via aqueous dispersion, comprising Eudragit ® L30D-55, and Polysorbate 80), and a second gastro-resistant (enteric coating) layer (coated via organic solution, comprising Eudragit ® L100-55, Polysorbate 80, and stearic acid).

### Examples:

### Example 1: Formulations A-C (comparison formulations)

### Formulation A:

This formulation comprises a core comprising a starter, and on said starter a coating layer comprising an acid-labile API (drug layer). Onto the drug layer, an intermediate layer (protection layer) for protecting the API was provided. Onto the intermediate layer, an enteric coating layer (gastro-resistant layer) coated via aqueous dispersion was provided (see Figure 1).

### Formulation B:

Formulation B comprises a core comprising a starter, and on said starter a coating layer comprising an acid-labile API (drug layer). Onto the drug layer, an intermediate layer (protection layer) for protecting the API was provided. Onto the intermediate layer, an enteric coating layer (gastro-resistant layer) coated via organic solution was provided (see Figure 2).

### Formulation C:

Formulation C comprises a core comprising a starter, and on said starter a coating layer comprising an acid-labile API (drug layer). Onto the drug layer, an intermediate layer (protection layer) for protecting the API was provided. Onto the intermediate layer, a first enteric coating layer (gastro-resistant layer) coated via aqueous dispersion was provided. Onto said first enteric coating layer a second enteric coating layer (gastro-resistant layer) is coated via organic solution (see Figure 3).

As a typical acid-labile API, esomeprazole was used as a "model" compound. It has to be understood that this model compound is only representative and illustrative for APIs in general, therefore not intended for limiting the scope of the claims in any way.

The characteristics of Formulations A-C are described in Table 1 below.

**Table 1:**

| | **Formulation A** | **Formulation B** | **Formulation C** |
|---|---|---|---|
| | (gastro-resistant layer: aqueous dispersion) | (gastro-resistant layer: organic solution) | (gastro-resistant layer: aqueous dispersion + organic solution) |
| **Formulation** | | | |
| **➢ Starter** | **identical quantitative composition** | | |
| **➢ Intermediate layer** | **identical quantitative composition** | | |
| **➢ Gastro-resistant layer** | **identical qualitative composition** | | |
| | *(except used solvent)* | | |
| **Stability of the API (in terms of degradation of the API over time** | A | ▼ | ▲ |
| **Level of gastro resistance of the API** | ▼ | ▲ | ▲ |
| **Process optimization** | | | |
| **➢ Workability** | ▼ | ▲ | ▲ |
| **➢ material inventory** | ▼ | ▲ | ▲ |

From table 1, it can be deduced that only Formulation C, comprising an intermediate layer coated via aqueous dispersion, followed by a coating having been applied via organic solution, shows both, enhanced stability (over the enteric coating) and gastro-resistance of the API.

### Example 2: Formulations B' (comparison formulation) and C' (formulation according to the present invention)

### Formulation B':

Formulation B' (Fig. 4) was prepared comprising a core comprising an acid-labile API. Onto the core, an intermediate layer for protecting the API was provided. Onto the intermediate layer, an enteric coating layer coated via organic solution was provided. The enteric coating layer comprised Eudragit ® L100-55, Polysorbate 80, propylene glycol, and stearic acid.

### Formulation C':

Formulation C' (Fig. 5) was prepared comprising a core comprising an acid-labile API. Onto the core, an intermediate layer for protecting the API was provided. Onto the intermediate layer, a first enteric coating layer coated via aqueous dispersion was provided. Onto said first enteric coating layer a second enteric coating layer was coated via organic solution. The first enteric coating layer comprised Eudragit ® L30D-55, and Polysorbate 80. The second enteric coating layer comprised Eudragit ® L100-55, Polysorbate 80, propylene glycol, and stearic acid. The characteristics of Formulations B' and C' are described in Table 2 below.

**Table 2:**

| | **Formulation B'** | | | **Formulation C'** | | |
|---|---|---|---|---|---|---|
| **Formulation** | | | | | | |
| **➢ Starter** | *identical* | | | *identical* | | |
| **➢ Intermediate layer** | *identical* | | | *identical* | | |
| **➢ Gastro-resistant layer** | *see description above* | | | *see description, above* | | |
| **Packaging material** | **HDPE³⁾ -bottles** | | | **HDPE³⁾-bottles** | | |
| **Stability *(sum of impurities in %) ¹⁾*** | release | month 1 | month 3 | release | month 1 | month 3 |
| | **0.22** | **1.15** | **3.43** | **0.05** | **0.29** | **0.89** |
| **Level of gastro-resistance *(%) ²⁾*** | release | month 1 | month 3 | release | month 1 | month 3 |
| | **63** | **84** | **78** | **63** | **87** | **79** |

| | | | | | | |
|---|---|---|---|---|---|---|
| *¹⁾ stability testing according ICH Q1AR2: accelerated conditions: 40°C* ± *2°C*/*75% RH* ± *5% RH* *²⁾ paddle, 100rpm, pretreatment: 4h 1000ml 0.05M Na-Acetate buffer pH 5.0* *³⁾ HDPE* = *High Density Polyethylene* | | | | | | |

As can be deduced from Table 2, the stability of the API is significantly enhanced (i.e. the sum of impurities of Formulation C' is significantly lower after 1 month and after 3 months compared to the sum of impurities of Formulation B' after 1 month and after 3 months). This improvement in stability of the API over the enteric coating as well as over the acidic environment in the stomach (gastro-resistance) can be attributed to the presence of a first and second enteric coating layer on the core comprising the acid-labile API.

### Example 3: Formulations B" and C" (comparison formulations)

### Formulation B":

Formulation B" (Fig. 6) was prepared comprising a core comprising an acid-labile API. Onto the core, an intermediate layer for protecting the API was provided. Onto the intermediate layer, an enteric coating layer coated via organic solution was provided. The enteric coating layer comprised Eudragit ®, L100-55, Polysorbate 80, and stearic acid.

### Formulation C":

Formulation C" (Fig. 7) was prepared comprising a core comprising an acid-labile API. Onto the core, an intermediate layer for protecting the API was provided. Onto the intermediate layer, a first enteric coating layer coated via aqueous dispersion was provided. Onto said first enteric coating layer a second enteric coating layer was coated via organic solution. The first enteric coating layer comprised Eudragit ® L30D-55, and Polysorbate 80. The second enteric coating layer comprised Eudragit ® L100-55, Polysorbate 80, and stearic acid.

The characteristics of Formulations B" and C" are described in Table 3 below:

**Table 3:**

| | **Formulation B"** | | | **Formulation C"** | | |
|---|---|---|---|---|---|---|
| **Formulation** | | | | | | |
| **➢ Starter** | *identical* | | | *identical* | | |
| **➢ Intermediate layer** | *identical* | | | *identical* | | |
| **➢ Gastro-resistant layer** | *see description above* | | | *see description above* | | |
| **Packaging material** | **HDPE³⁾-bottles** | | | **HDPE³⁾-bottles** | | |
| **Stability** *(sum of impurities in %)¹⁾* | release | month 1 | month 3 | release | month 1 | month 3 |
| | **0.32** | **0,80** | **2.10** | **0.27** | **0.70** | **1.97** |
| **Level of gastro-resistance** *(%) ²⁾* | release | month 1 | month 3 | release | month 1 | month 3 |
| | **90** | **90** | **89** | **93** | **91** | **89** |

| | | | | | | |
|---|---|---|---|---|---|---|
| *¹⁾ stability testing according ICH Q1AR2: accelerated conditions: 40°C* ± 2°*C*/*75% RH* ± 5% *RH* *²⁾ paddle, 100rpm, pretreatment: 4h 1000ml 0.05M Na-Acetate buffer pH 5.0* *³⁾ HDPE = High Density Polyethylene* | | | | | | |

As can be deduced from Table 3, the stability of the API is significantly enhanced (i.e. the sum of impurities of Formulation C" is lower after 1 month and after 3 months compared to the sum of impurities of Formulation B" after 1 month and after 3 months). This improvement in stability of the API over the enteric coating as well as over the acidic environment in the stomach (gastro-resistance) can be attributed to the presence of a first and second enteric coating layer on the core comprising the acid-labile API.

## Claims

1. A process for the preparation of a coated particle comprising the steps of:
i) providing a core comprising at least one active pharmaceutical ingredient (API),
ii) providing an intermediate layer onto said core,
iii) providing a first enteric coating layer onto said intermediate layer by applying an aqueous dispersion of an enteric polymer onto said intermediate layer,
iv) providing a second enteric coating layer onto said first enteric coating layer by applying an organic solution of an enteric polymer onto said first enteric layer, wherein said organic solution comprises one or more fatty acids and one or more emulsifiers and hydrophilic plasticizer, wherein said hydrophilic plasticizer is propylene glycol, and
v) obtaining said coated particle,
wherein the enteric polymer used in step iii) and iv) is an anionic polymer or copolymer.

2. The process according to claim 1, wherein the core comprises at least one acid-labile API.

3. The process according to claim 1 or 2, wherein at least one API in the core is selected from the group consisting of pravastatin, fluvastatin, atorvastatin, penicillin G, ampicillin, streptomycin, clarithromycin, azithromycin, dideoxyinosine, dideoxyadenosine, dideoxycytosine, digoxin, pancreatin, bupropion, lansoprazole, omeprazole, esomeprazole, pantoprazole, rabeprazole, and leminoprazole, hydroxyomeprazole, pariprazole, perprazole, tenatoprazole, diclofenac and pharmaceutically acceptable salts thereof, preferably at least one API is esomeprazole or a pharmaceutically acceptable salt thereof, and more preferably the API is esomeprazole or a pharmaceutically acceptable salt thereof.

4. The process according to any of the preceding claims, wherein the intermediate layer does not contain an enteric polymer.

5. The process according to any of the preceding claims, wherein in step iii) the amount of enteric polymer dispersion is in the range of 0.5-20 %, more preferably 5-15 %, based on the weight of the core, and wherein in step iv) the amount of enteric polymer solution for the second enteric coating layer is in the range of 10-100 %, more preferably 20-60 %, based on the weight of the core, and/or wherein in step iii) the concentration of enteric polymer in the aqueous dispersion is in the range of 10-40 %, preferably 20-30 %, based on the weight of the aqueous polymer dispersion, and wherein in step iv) the concentration of enteric polymer in the organic solution is in the range of 1-20 %, preferably 8-12 %, based on the weight of the organic polymer solution.

6. The process according to any of the preceding claims, wherein the core is selected from the group consisting of
i) non-pareilles coated with at least one layer comprising the at least one API; optionally, the non-pareilles is additionally coated with a further layer comprising a further API;
ii) pellets or granules comprising the at least one API, wherein the pellets or granules are obtained by granulation or extrusion processes; optionally, the pellets or granules additionally comprise a further API; and
iii) pellets or granules comprising the at least one API, wherein the pellets or granules are obtained by granulation or extrusion processes and additionally comprise one or more coated layers comprising one or more further APIs.

7. The process according to any of the preceding claims, wherein the coated particle comprises only two enteric coating layers in total.

8. A process for the preparation of a dosage form, comprising
a) process for the preparation of a coated particle according to any of claims 1 to 7, and
b) formulating the coated particles of a) into a dosage form.

9. A coated particle, obtainable and/or obtained according to any one of the processes of claims 1-7.

10. A dosage form, obtainable and/or obtained according to claim 8.

11. A coated particle comprising, from the inside to the outside:
i) a core comprising at least one active pharmaceutical ingredient (API),
ii) an intermediate layer on said core,
iii) a first enteric coating layer comprising an enteric polymer or combinations of enteric polymers on said intermediate layer,
iv) a second enteric coating layer comprising an enteric polymer or combinations of enteric polymers on said first enteric coating layer, wherein said second enteric coating layer comprises one or more fatty acids and one or more emulsifiers and hydrophilic plasticizer, wherein said hydrophilic plasticizer is propylene glycol,
wherein the second enteric coating layer differs from the first enteric coating layer
(a) in terms of different types of residual solvent that is respectively present in the first and in the second enteric coating layer, the different types of residual solvent being defined by, in comparison with the other type, a relatively higher amount of organic residual solvent in the second enteric coating layer and a relatively higher amount of water residual solvent in the first enteric coating layer, and/or
b) in terms of a different compactness of the first and the second enteric coating layer, wherein the first enteric coating layer is less compact than the second enteric coating layer, provided that the enteric polymer/s in the first and in the second enteric coating layer are the same, and/or
c) in terms of a different density of the first and the second enteric coating layer, wherein the first enteric coating layer is less dense than the second enteric coating layer, provided that the enteric polymer/s in the first and in the second enteric coating layer are the same,
wherein the enteric polymer comprised in said first and second enteric coating layer is an anionic polymer or copolymer.

12. The coated particle according to claim 11, **characterized by** any one or a combination of the following features:
the core is as defined in claim 6;
the API is as defined in claim 3;
the intermediate layer is as defined in claim 4;
the enteric polymer in the first and/or second enteric coating layer is an anionic polymer or copolymer, preferably an anionic polymer or copolymer with methacrylic acid as functional group;
the first enteric coating layer further comprises an emulsifier;
the second enteric coating layer comprises one or more fatty acids and one or more emulsifiers and hydrophilic plasticizer, wherein said hydrophilic plasticizer is propylene glycol.

13. A dosage form comprising coated particles according to claims 11 or 12.

14. Use of a dosage form according to claim 10, or according to claim 13, as a medicament.

15. Use of a combination of
a first enteric coating layer formed from an aqueous dispersion comprising an enteric polymer, and
a second enteric coating layer formed on top of the first enteric coating layer and being formed from an organic solution of an enteric polymer having acidic groups, wherein said organic solution comprises one or more fatty acids and one or more emulsifiers and hydrophilic plasticizer, wherein said hydrophilic plasticizer is propylene glycol,
for the stabilization of an acid-labile API present in a compartment formed underneath the first enteric coating layer, with optional intermediate layer(s) in between said compartment and said first enteric coating layer,
wherein the enteric polymer is an anionic polymer or copolymer.

## Patentansprüche

1. Verfahren zur Herstellung eines beschichteten Partikels, umfassend die Schritte:
i) Bereitstellen eines Kerns umfassend mindestens einen pharmazeutisch aktiven Wirkstoff (API),
ii) Bereitstellen einer Zwischenschicht auf diesen Kern,
iii) Bereitstellen einer ersten enterischen Deckschicht auf diese Zwischenschicht durch Aufbringen einer wässrigen Dispersion eines enterischen Polymers auf diese Zwischenschicht,
iv) Bereitstellen einer zweiten enterischen Deckschicht auf die erste enterische Deckschicht durch Aufbringen einer organischen Lösung eines enterischen Polymers auf die erste enterische Schicht, wobei die organische Lösung eine oder mehrere Fettsäuren und einen oder mehrere Emulgatoren und hydrophile Weichmacher umfasst, wobei der hydrophile Weichmacher Propylenglykol ist, und
v) Erhalten des beschichteten Partikels,
wobei das in Schritt iii) und iv) verwendete enterische Polymer ein anionisches Polymer oder Copolymer ist.

2. Verfahren gemäß Anspruch 1, wobei der Kern mindestens einen säure-labilen API umfasst.

3. Verfahren gemäß Anspruch 1 oder 2, wobei der mindestens eine API in dem Kern ausgewählt ist aus der Gruppe bestehend aus Pravastatin, Fluvastatin, Atorvastatin, Penicillin G, Ampicillin, Streptomycin, Clarithromycin, Azithromycin, Dideoxyinosin, Dideoxyadenosin, Dideoxycytosin, Digoxin, Pancreatin, Bupropion, Lansoprazol, Omeprazol, Esomeprazol, Pantoprazol, Rabeprazol und Leminoprazol, Hydroxyomeprazol, Pariprazol, Perprazol, Tenatoprazol, Diclofenac und pharmazeutisch annehmbare Salze davon, bevorzugt ist ein API Esomeprazol oder ein pharmazeutisch annehmbares Salz davon, und mehr bevorzugt ist das API Esomeprazol oder ein pharmazeutisch annehmbares Salz davon.

4. Verfahren gemäß irgendeinem der vorhergehenden Ansprüche, wobei die Zwischenschicht kein enterisches Polymer enthält.

5. Verfahren gemäß irgendeinem der vorhergehenden Ansprüche, wobei in Schritt iii) die Menge der enterischen Polymerdispersion im Bereich von 0,5 - 20%, mehr bevorzugt 5 - 15%, basierend auf dem Gewicht des Kerns, liegt, und wobei in Schritt iv) die Menge der enterischen Polymerlösung für die zweite enterische Deckschicht im Bereich von 10 - 100%, mehr bevorzugt 20 - 60%, basierend auf dem Gewicht des Kerns, liegt, und/oder wobei in Schritt iii) die Konzentration des enterischen Polymers in der wässrigen Dispersion in dem Bereich von 10 - 40%, bevorzugt 20 - 30%, basierend auf dem Gewicht der wässrigen Polymerdispersion, liegt, und wobei in Schritt iv) die Konzentration des enterischen Polymers in der organischen Lösung in dem Bereich von 1 - 20%, bevorzugt 8 - 12%, basierend auf dem Gewicht der organischen Polymerlösung, liegt.

6. Verfahren gemäß irgendeinem der vorhergehenden Ansprüche, wobei der Kern ausgewählt ist aus der Gruppe bestehend aus
i) Non-Pareilles, beschichtet mit mindestens einer Schicht umfassend den mindestens einen API; optional ist der Non-Pareille zusätzlich mit einer weiteren Schicht umfassend einen weiteren API beschichtet;
ii) Pellets oder Granulate umfassend den mindestens einen API, wobei die Pellets oder die Granulate erhalten werden durch Granulierung oder Extrusions-Verfahren; optional umfassen die Pellets oder Granulate zusätzlich einen weiteren API; und
iii) Pellets oder Granulate umfassend den mindestens einen API, wobei die Pellets oder die Granulate erhalten werden durch Granulierung oder Extrusions-Verfahren und zusätzlich eine oder mehrere Deckschichten, umfassend einen oder mehrere weitere APIs, umfassen.

7. Verfahren gemäß irgendeinem der vorhergehenden Ansprüche, wobei der beschichtete Partikel nur zwei enterische Deckschichten insgesamt umfasst.

8. Verfahren zur Herstellung einer Darreichungsform, umfassend
a) Verfahren zur Herstellung eines beschichteten Partikels gemäß irgendeinem der Ansprüche 1 bis 7, und
b) Formulieren des beschichteten Partikels von a) in eine Darreichungsform.

9. Beschichteter Partikel, erhältlich und/oder erhalten gemäß irgendeinem der Verfahren der Ansprüche 1 bis 7.

10. Darreichungsform, erhältlich und/oder erhalten gemäß Anspruch 8.

11. Beschichteter Partikel umfassend, von innen nach außen:
i) Kern, umfassend mindestens einen pharmazeutisch aktiven Wirkstoff (API),
ii) eine Zwischenschicht auf dem Kern,
iii) eine erste enterische Deckschicht umfassend ein enterisches Polymer oder Kombinationen von enterischen Polymeren auf der Zwischenschicht,
iv) eine zweite enterische Deckschicht umfassend ein enterisches Polymer oder Kombinationen von enterischen Polymeren auf der ersten enterischen Deckschicht, wobei die zweite enterische Deckschicht ein oder mehrere Fettsäuren umfasst und einen oder mehrere Emulgatoren und hydrophile Weichmacher, wobei der hydrophile Weichmacher Propylenglykol ist,
wobei sich die zweite enterische Deckschicht von der ersten enterischen Deckschicht unterscheidet
(a) hinsichtlich verschiedener Arten von restlichem Lösungsmittel, das jeweils in der ersten und in der zweiten enterischen Deckschicht vorhanden ist, die verschiedenen Arten von restlichem Lösungsmittel sind, im Vergleich mit der anderen Art, definiert durch eine vergleichsweise größere Menge von restlichem organischem Lösungsmittel in der zweiten enterischen Deckschicht und einer vergleichsweise höheren Menge von restlichem wässrigen Lösungsmittel in der ersten enterischen Beschichtungsschicht, und/oder
b) hinsichtlich einer unterschiedlichen Kompaktheit der ersten und der zweiten enterischen Deckschicht, wobei die erste enterische Deckschicht weniger kompakt ist als die zweite enterische Deckschicht, vorausgesetzt, dass das/die enterische(n) Polymer(e) in der ersten und in der zweiten enterischen Deckschicht die gleichen sind, und/oder
c) hinsichtlich einer unterschiedlichen Dichte der ersten und der zweiten enterischen Deckschicht, wobei die erste enterische Deckschicht weniger dicht ist als die zweite enterische Deckschicht, vorausgesetzt, dass
das/die enterische(n) Polymer(e) in der ersten und in
der zweiten enterischen Deckschicht die gleichen sind, wobei das enterische Polymer in der ersten und zweiten enterischen Deckschicht ein anionisches Polymer oder Copolymer umfasst.

12. Beschichteter Partikel gemäß Anspruch 11, **gekennzeichnet durch** irgendeines oder eine Kombination der folgenden Merkmale:
der Kern ist wie in Anspruch 6 definiert;
der API ist wie in Anspruch 3 definiert;
die Zwischenschicht ist wie in Anspruch 4 definiert;
das enterische Polymer in der ersten und/oder zweiten enterischen Deckschicht ist ein anionisches Polymer oder Copolymer, bevorzugt ein anionisches Polymer oder Copolymer mit Methacryl-Säure als funktionelle Gruppe;
die erste enterische Deckschicht umfasst ferner einen Emulgator;
die zweite enterische Deckschicht umfasst eine oder mehrere Fettsäuren und einen oder mehrere Emulgatoren und hydrophilen Weichmacher, wobei der hydrophile Weichmacher Propylenglykol ist.

13. Darreichungsform umfassend beschichtete Partikel gemäß der Ansprüche 11 oder 12.

14. Verwendung einer Darreichungsform gemäß Anspruch 10 oder gemäß Anspruch 13, als Medikament.

15. Verwendung einer Kombination von
einer ersten enterischen Deckschicht gebildet aus einer wässrigen Dispersion umfassend ein enterisches Polymer, und
einer zweiten enterischen Deckschicht gebildet auf der ersten enterischen Deckschicht und geformt aus einer organischen Lösung eines enterischen Polymers mit Säuregruppen, wobei die organische Lösung ein oder mehrere Fettsäuren und einen oder mehrere Emulgatoren und hydrophilen Weichmacher umfasst, wobei der hydrophile Weichmacher Propylenglykol ist,
zur Stabilisierung eines säure-labilen API, der in einem Kompartiment vorhanden ist, welches unter der ersten enterischen Deckschicht gebildet ist, mit optionalen Zwischenschicht(en) zwischen diesem Kompartiment und dieser ersten enterischen Deckschicht,
wobei das enterische Polymer ein anionisches Polymer oder Copolymer ist.

## Revendications

1. Procédé de préparation d'une particule enrobée, comprenant les étapes qui consistent à :
i) fournir un noyau comprenant au moins un principe actif pharmaceutique (PAP),
ii) former une couche intermédiaire sur ledit noyau,
iii) former une première couche de revêtement gastro-résistant sur ladite couche intermédiaire par application d'une dispersion aqueuse d'un polymère gastro-résistant sur ladite couche intermédiaire,
iv) former une deuxième couche de revêtement gastro-résistant sur ladite première couche de revêtement gastro-résistant par application d'une solution organique d'un polymère gastro-résistant sur ladite première couche gastro-résistante, ladite solution organique comprenant un ou plusieurs acides gras et un ou plusieurs émulsifiants et un plastifiant hydrophile, ledit plastifiant hydrophile étant le propylène glycol, et
v) obtenir ladite particule enrobée,
dans lequel le polymère gastro-résistant utilisé dans les étapes iii) et iv) est un polymère ou un copolymère anionique.

2. Procédé selon la revendication 1, dans lequel le noyau comprend au moins un PAP instable aux acides.

3. Procédé selon la revendication 1 ou 2, dans lequel au moins un PAP dans le noyau est sélectionné dans le groupe constitué de la pravastatine, de la fluvastatine, de l'atorvastatine, de la pénicilline G, de l'ampicilline, de la streptomycine, de la clarithromycine, de l'azithromycine, de la didéoxyinosine, de la didéoxyadénosine, de la didéoxycytosine, de la digoxine, de la pancréatine, du bupropion, du lansoprazole, de l'oméprazole, de l'ésoméprazole, du pantoprazole, du rabéprazole, et du léminoprazole, de l'hydroxyoméprazole, du pariprazole, du perprazole, du ténatoprazole, du diclofénac et de sels pharmaceutiquement acceptables de ceux-ci, préférablement au moins un PAP est l'ésoméprazole ou un sel pharmaceutiquement acceptable de celui-ci, et plus préférablement le PAP est l'ésoméprazole ou un sel pharmaceutiquement acceptable de celui-ci.

4. Procédé selon l'une quelconque des revendications précédentes, dans lequel la couche intermédiaire ne contient pas de polymère gastro-résistant.

5. Procédé selon l'une quelconque des revendications précédentes, dans lequel dans l'étape iii) la quantité de dispersion de polymère gastro-résistant est de 0,5 % à 20 %, plus préférablement de 5 % à 15 %, relativement au poids du noyau, et dans lequel dans l'étape iv) la quantité de solution de polymère gastro-résistant pour la deuxième couche de revêtement gastro-résistant est de 10 % à 100 %, plus préférablement de 20 % à 60 %, relativement au poids du noyau, et/ou dans lequel dans l'étape iii) la concentration de polymère gastro-résistant dans la dispersion aqueuse est de 10 % à 40 %, préférablement de 20 % à 30 %, relativement au poids de la dispersion aqueuse de polymère, et dans lequel dans l'étape iv) la concentration de polymère gastro-résistant dans la solution organique est de 1 % à 20 %, préférablement de 8 % à 12 %, relativement au poids de la solution organique de polymère.

6. Procédé selon l'une quelconque des revendications précédentes, dans lequel le noyau est sélectionné dans le groupe constitué de :
i) non-pareils revêtus d'au moins une couche comprenant ledit au moins un PAP ; optionnellement, les non-pareils sont en outre revêtus d'une couche supplémentaire comprenant un PAP supplémentaire ;
ii) pastilles ou granulés comprenant ledit au moins un PAP, les pastilles ou les granulés étant obtenus par des procédés d'agglomération en granulés ou d'extrusion ; optionnellement, les pastilles ou les granulés comprennent en outre un PAP supplémentaire ; et
iii) pastilles ou granulés comprenant ledit au moins un PAP, les pastilles ou les granulés étant obtenus par des procédés d'agglomération en granulés ou d'extrusion et comprenant en outre une ou plusieurs couches appliquées de façon à former des revêtements comprenant un ou plusieurs PAP supplémentaires.

7. Procédé selon l'une quelconque des revendications précédentes, dans lequel la particule enrobée comprend seulement deux couches de revêtements gastro-résistants au total.

8. Procédé de préparation d'une forme pharmaceutique, comprenant :
a) un procédé de préparation d'une particule enrobée selon l'une quelconque des revendications 1 à 7, et
b) une formulation intégrant les particules enrobées de a) dans une forme pharmaceutique.

9. Particule enrobée, pouvant être obtenue et/ou obtenue par l'un quelconque des procédés selon les revendications 1 à 7.

10. Forme pharmaceutique, pouvant être obtenue et/ou obtenue selon la revendication 8.

11. Particule enrobée comprenant, de l'intérieur vers l'extérieur :
i) un noyau comprenant au moins un principe actif pharmaceutique (PAP),
ii) une couche intermédiaire sur ledit noyau,
iii) une première couche de revêtement gastro-résistant comprenant un polymère gastro-résistant ou des combinaisons de polymères gastro-résistants sur ladite couche intermédiaire,
iv) une deuxième couche de revêtement gastro-résistant comprenant un polymère gastro-résistant ou des combinaisons de polymères gastro-résistants sur ladite première couche de revêtement gastro-résistant, ladite deuxième couche de revêtement gastro-résistant comprenant un ou plusieurs acides gras et un ou plusieurs émulsifiants et un plastifiant hydrophile, ledit plastifiant hydrophile étant le propylène glycol,
la deuxième couche de revêtement gastro-résistant différant de la première couche de revêtement gastro-résistant
(a) par différents types de solvants résiduels qui sont présents, respectivement, dans la première couche de revêtement gastro-résistant et dans la deuxième couche de revêtement gastro-résistant, les différents types de solvants résiduels étant définis par, en comparaison avec l'autre type, une quantité relativement supérieure de solvant résiduel organique dans la deuxième couche de revêtement gastro-résistant et une quantité relativement supérieure de solvant résiduel aqueux dans la première couche de revêtement gastro-résistant, et/ou
(b) par une différente compacité de la première couche de revêtement gastro-résistant et de la deuxième couche de revêtement gastro-résistant, la première couche de revêtement gastro-résistant étant moins compacte que la deuxième couche de revêtement gastro-résistant, sous réserve que le(s) polymère(s) gastro-résistant(s) dans la première couche de revêtement gastro-résistant et dans la deuxième couche de revêtement gastro-résistant soient identiques, et/ou
(c) par une différente densité de la première couche de revêtement gastro-résistant et de la deuxième couche de revêtement gastro-résistant, la première couche de revêtement gastro-résistant étant moins dense que la deuxième couche de revêtement gastro-résistant, sous réserve que le(s) polymère(s) gastro-résistant(s) dans la première couche de revêtement gastro-résistant et dans la deuxième couche de revêtement gastro-résistant soient identiques,
dans laquelle le polymère gastro-résistant présent dans ladite première couche de revêtement gastro-résistant et dans ladite deuxième couche de revêtement gastro-résistant est un polymère ou un copolymère anionique.

12. Particule enrobée selon la revendication 11, **caractérisée par** l'une quelconque ou par une combinaison des caractéristiques suivantes :
le noyau est tel que défini dans la revendication 6 ;
le PAP est tel que défini dans la revendication 3 ;
la couche intermédiaire est telle que définie dans la revendication 4 ;
le polymère gastro-résistant dans la première couche de revêtement gastro-résistant et/ou la deuxième couche de revêtement gastro-résistant est un polymère ou un copolymère anionique, préférablement un polymère ou un copolymère anionique comportant de l'acide méthacrylique comme groupe fonctionnel ;
la première couche de revêtement gastro-résistant comprend en outre un émulsifiant ;
la deuxième couche de revêtement gastro-résistant comprend un ou plusieurs acides gras et un ou plusieurs émulsifiants et un plastifiant hydrophile, ledit plastifiant hydrophile étant le propylène glycol.

13. Forme pharmaceutique comprenant des particules enrobées selon les revendications 11 ou 12.

14. Utilisation d'une forme pharmaceutique selon la revendication 10, ou selon la revendication 13, comme médicament.

15. Utilisation d'une combinaison de :
une première couche de revêtement gastro-résistant formée à partir d'une dispersion aqueuse comprenant un polymère gastro-résistant, et
une deuxième couche de revêtement gastro-résistant formée sur le dessus de la première couche de revêtement gastro-résistant et formée à partir d'une solution organique d'un polymère gastro-résistant comportant des groupes acides,
dans laquelle ladite solution organique comprend un ou plusieurs acides gras et un ou plusieurs émulsifiants et un plastifiant hydrophile, ledit plastifiant hydrophile étant le propylène glycol,
pour la stabilisation d'un PAP instable aux acides présent dans un compartiment formé en-dessous de la première couche de revêtement gastro-résistant, une ou plusieurs couches intermédiaires optionnelles étant présentes entre ledit compartiment et ladite première couche de revêtement gastro-résistant,
dans laquelle le polymère gastro-résistant est un polymère ou un copolymère anionique.
